# EUROPEAN PATENT APPLICATION

(11) **EP 1 125 928 A1**
(43) Date of publication of application: **22.08.2001**
(21) Application number: 00956837.9
(22) Date of filing: 31.08.2000
(51) Int. Cl.: C07D 213/53, C08F 4/70, C08F 10/00

(54) **TRANSITION METAL COMPOUNDS, CATALYSTS FOR THE PRODUCTION OF ALPHA-OLEFINS AND PROCESS THEREFOR**

(30) Priority: 02.09.1999 JP 24901299; 16.09.1999 JP 26256499; 17.09.1999 JP 26309399
(71) Applicant: IDEMITSU PETROCHEMICAL CO. Ltd., Tokyo 130-0015 (JP)
(72) Inventor: WATANABE, Masami, Ichihara-shi, Chiba 299-0107 (JP); SATO, Haruhito, Ichihara-shi, Chiba 299-0107 (JP); KURAMOTO, Masahiko, Ichihara-shi, Chiba 299-0107 (JP); TASHIRO, Hironori, Tokuyama-shi, Yamaguchi 745-0843 (JP); TANAKA, Shinji, Tokuyama-shi, Yamaguchi 745-0843 (JP); TAMURA, Takao, Tokuyama-shi, Yamaguchi 745-0843 (JP)
(74) Representative: Gille Hrabal Struck Neidlein Prop Roos
(86) International application number: JP0005913
(87) International publication number: WO0117967

(57) **Abstract**

A transition metal compound represented by the general formula (1): 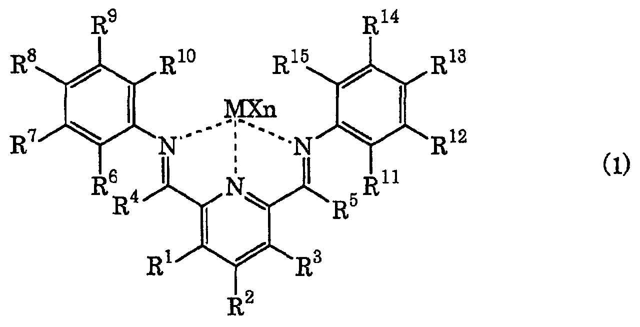 or the general formula (2): 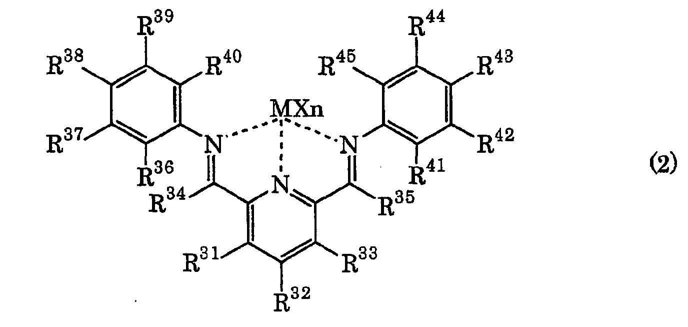 wherein M, R¹ to R¹⁵, R³¹ to R⁴⁵, X and n are as defined in the specification. By using a catalyst comprising (A) the transition metal compound of the formula (1) or (2) containing a transition metal selected from the group consisting of elements of Groups 8 to 10 of the Periodic Table; (B) at least one compound selected from the group consisting of an organoaluminum compound (B-1), an ionic compound (B-2) capable of converting the transition metal compound into a cationic transition metal compound, a Lewis acid (B-3), and clay, clay mineral and an ion-exchangeable layer compound (B-4); and (C) an optional organometallic compound, α-olefins are efficiently produced with a less amount of by-products such as heavy components and waxes.

## Description

### TECHNICAL FIELD

The present invention relates to a transition metal compound, a catalyst for the production of α-olefins and a process for producing α-olefins. More particularly, the present invention relates to a transition metal compound suitable for forming a catalyst for the production of α-olefins which is capable of minimizing by-products such as heavy components and waxes upon the production of α-olefins, such a catalyst for the production of α-olefins and a process for producing α-olefins.

### BACKGROUND ARTS

Hitherto, as the method for producing ethylene oligomers (also called "α-olefins") by the polymerization of ethylene, there is known the process using a nickel complex (Shell Higher Olefin Process: SHOP). However, this method has such a problem that the catalytic activity of the complex is low. Recently, it has been found that a nickel-diimine complex (International Patent Publication 96/23010), or a chelate complex of iron or cobalt (Chem. Commun., 1988, pp. 849-850; J. Am. Chem. Soc., 1998, 120, pp. 7143-7144, and J. Am. Chem. Soc., 1998, 120, pp. 4049-4050), exhibits a high catalytic activity to the polymerization of ethylene. For example, as reported in the prior arts, in the production processes using an iron chelate complex as a main catalyst and a methyl aluminoxane as a co-catalyst, not only a high catalytic activity to the polymerization of ethylene but also a high selectivity to terminal groups of ethylene oligomers produced, have been attained. Processes for the polymerization of ethylene using similar complexes have also been disclosed in International Patent Publication 98/27124, 99/02472 and 99/12981. However, any of the conventional processes has drawbacks such as the use of a large amount of expensive aluminoxane, the production of internal olefins as main products, and the production of a large amount of by-product polymers. At the present time, any conventional process for the production of α-olefins have failed to not only show an excellent catalytic activity per unit weight of catalyst used, but also reduce amounts of by-products such as heavy components and wax components.

### DISCLOSURE OF THE INVENTION

The present invention has been made in view of the above problems. An object of the present invention is to provide a transition metal compound suitable for forming a catalyst for the production of α-olefins which is capable of minimizing by-products such as heavy components and waxes upon the production of α-olefins, and shows a high activity to the oligomerization of ethylene, such a catalyst for the production of α-olefins, and a process for producing α-olefins.

The present inventors have found that a specific transition metal compound containing a transition metal selected from the group consisting of elements of Groups 8 to 10 of the Periodic Table, and a catalyst for the production of α-olefins comprising the transition metal compound and at least one compound selected from the group consisting of an organoaluminum compound, an ionic compound, a Lewis acid, and clay, clay mineral and an ion-exchangeable layer compound, effectively accomplish the above objects. The present invention has been completed based on this finding.

Namely, in a first aspect of the present invention, there is provided a transition metal compound represented by the general formula (1): 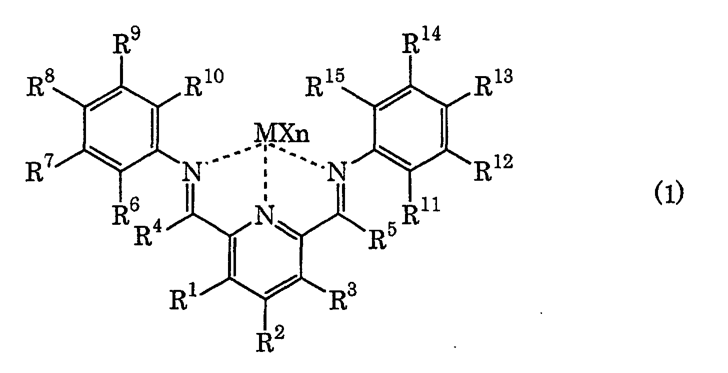 wherein M is a transition metal selected from the group consisting of elements of Groups 8 to 10 of the Periodic Table; R¹ to R³ are independently a hydrogen atom, a halogen atom, a C₁-C₂₀ hydrocarbon group, a C₁-C₂₀ halogenated hydrocarbon group or a hetero atom-containing group, and may be bonded to each other to form a ring; R⁴ and R⁵ are a hydrogen atom or a C₁-C₂₀ hydrocarbon group with the proviso that at least one of R⁴ and R⁵ represents a hydrogen atom, aryl or substituted aryl; R⁶ to R¹⁵ are independently a hydrogen atom, a halogen atom, a C₁-C₂₀ hydrocarbon group or a hetero atom-containing group with the proviso that at least one of R⁶ and R¹⁰ or at least one of R¹¹ and R¹⁵ is a hydrogen atom, and R⁶ to R¹⁵ may be bonded to each other to form a ring; X is a covalent or ionic bonding group, and when a plurality of X groups are present, these groups may be the same or different; and n is a valence of M.

In a second aspect of the present invention, there is provided a transition metal compound represented by the general formula (2): 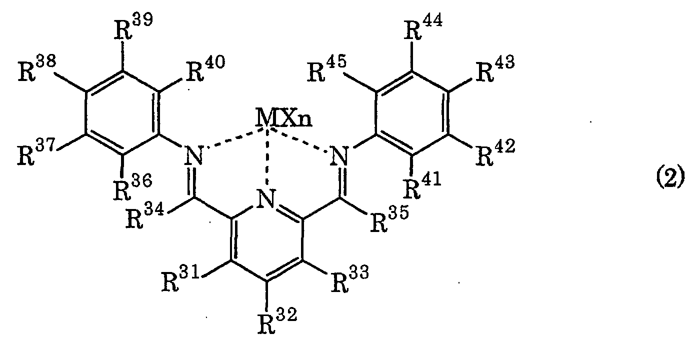 wherein M is a transition metal selected from the group consisting of elements of Groups 8 to 10 of the Periodic Table; R³¹ to R³³ are independently a hydrogen atom, a halogen atom, a C₁-C₂₀ hydrocarbon group, a C₁-C₂₀ halogenated hydrocarbon group or a hetero atom-containing group; R³⁴ and R³⁵ are independently a hydrogen atom or a C₁-C₂₀ hydrocarbon group; R⁴⁰ and R⁴⁵ are a C₁-C₂₀ hydrocarbon group; R³⁶ and R⁴¹ are a hydrogen atom; R³⁷ to R³⁹ are independently a hydrogen atom, a halogen atom or a C₁-C₂₀ hydrocarbon group with the proviso that at least one of R³⁷ to R³⁹ represents a halogen atom; R⁴² to R⁴⁴ are independently a hydrogen atom, a halogen atom or a C₁-C₂₀ hydrocarbon group with the proviso that at least one of R⁴² to R⁴⁴ represents a halogen atom; R³¹ to R³⁵ or R³⁶ to R⁴⁵ may be bonded to each other to form a ring; X is a covalent or ionic bonding group, and when a plurality of X groups are present, these groups may be the same or different; and n is a valence of M.

In a third aspect of the present invention, there is provided a catalyst for the production of α-olefins, comprising:
(A) any of the above transition metal compounds; and
(B) at least one compound selected from the group consisting of an organoaluminum compound (B-1), an ionic compound (B-2) capable of converting the transition metal compound into a cationic transition metal compound when reacted therewith, a Lewis acid (B-3), and clay, clay mineral and an ion-exchangeable layer compound (B-4).

In a fourth aspect of the present invention, there is provided a process for producing α-olefins by polymerizing ethylene in the presence of any of the above catalysts for the production of α-olefins.

### BRIEF DESCRIPTION OF THE DRAWINGS

Fig. 1 is a ¹H-NMR chart of 2,6-diacetylpyridine-bis(4-fluoro-2-tolylimine) as a ligand prepared in EXAMPLE 23.
Fig. 2 is a ¹H-NMR chart of 2,6-diacetylpyridine-bis(5-fluoro-2-tolylimine) as a ligand prepared in EXAMPLE 24.
Fig. 3 is a ¹H-NMR chart of 2,6-diacetylpyridine-bis(3-fluoro-2-tolylimine) as a ligand prepared in EXAMPLE 25.
Fig. 4 is a ¹H-NMR chart of 2,6-pyridinedicarboxyaledhyde-bis(4-fluoro-2-tolylimine) as a ligand prepared in EXAMPLE 26.
Fig. 5 is a ¹H-NMR chart of 2,6-pyridinedicarboxyaledhyde-bis(5-fluoro-2-tolylimine) as a ligand prepared in EXAMPLE 27.
Fig. 6 is a ¹H-NMR chart of 2,6-pyridinedicarboxyaledhyde-bis(3-fluoro-2-tolylimine) as a ligand prepared in EXAMPLE 28.

### PREFERRED EMBODIMENT TO CARRY OUT THE INVENTION

The α-olefins (oligomers) used herein include polymers having a molecular weight of 10,000 or lower and containing vinyl groups at terminals thereof, and are different in properties and applications from ordinary ethylene polymers having a higher molecular weigh and showing inherent properties of high-molecular weight polymers. Therefore, properties of a catalyst used for the production of these α-olefins are inevitably different from those of catalysts used for the production of ordinary high-molecular weight polymers. Thus, the catalysts for the production of ordinary high-molecular weight polymers are not necessarily usable for the production of the α-olefins.

### [1] Transition metal compound

The first transition metal compound of the present invention is a Group 8 to 10 transition metal compound represented by the general formula (1): 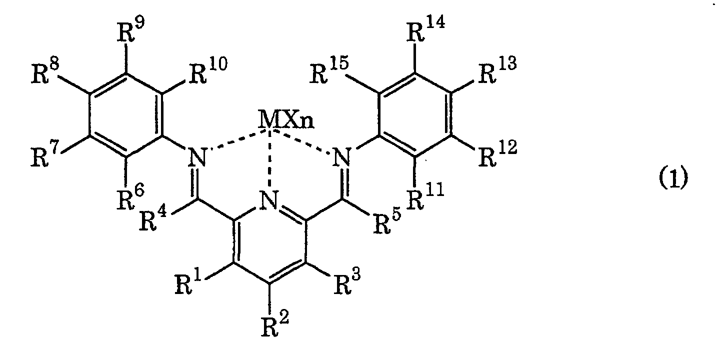 wherein M is a transition metal selected from the group consisting of elements of Groups 8 to 10 of the Periodic Table; R¹ to R³ are independently a hydrogen atom, a halogen atom, a C₁-C₂₀ hydrocarbon group, a C₁-C₂₀ halogenated hydrocarbon group or a hetero atom-containing group, and may be bonded to each other to form a ring; R⁴ and R⁵ are a hydrogen atom or a C₁-C₂₀ hydrocarbon group with the proviso that at least one of R⁴ and R⁵ represents a hydrogen atom, aryl or substituted aryl; R⁶ to R¹⁵ are independently a hydrogen atom, a halogen atom, a C₁-C₂₀ hydrocarbon group or a hetero atom-containing group with the proviso that at least one of R⁶ and R¹⁰ or at least one of R¹¹ and R¹⁵ is a hydrogen atom, and R⁶ to R¹⁵ may be bonded to each other to form a ring; X is a covalent or ionic bonding group, and when a plurality of X groups are present, these groups may be the same or different; and n is a valence of M.

In the general formula (1), M represents a transition metal selected from the group consisting of elements of Groups 8 to 10 of the Periodic Table. Of these transition metals, preferred are iron and cobalt.

R¹ to R³ independently represent a hydrogen atom, a halogen atom, a C₁-C₂₀ hydrocarbon group, a C₁-C₂₀ halogenated hydrocarbon group or a hetero atom-containing group, and may be bonded to each other to form a ring. The halogen atoms include fluorine, chlorine, bromine and iodine. The C₁-C₂₀ hydrocarbon groups include, for example, C₁-C₂₀ linear or branched alkyl, C₃-C₂₀ cycloalkyl, C₆-C₂₀ aryl and C₇-C₂₀ arylalkyl. Specific examples of the C₁-C₂₀ linear or branched alkyl groups include methyl, ethyl, n-propyl, isopropyl, n-butyl, isobutyl, sec-butyl, tert-butyl, various pentyl groups, various hexyl groups, various octyl groups, various decyl groups, various tetradecyl groups, various hexadecyl groups and various octadecyl groups. Specific examples of the C₃-C₂₀ cycloalkyl groups include cyclopentyl, cyclohexyl and cyclooctyl wherein an appropriate substitutent(s) such as lower alkyl may be bonded to the cycloalkyl ring. Specific examples of the C₆-C₂₀ aryl groups include phenyl, tolyl, xylyl, naphthyl and methylnaphthyl. Specific examples of the C₇-C₂₀ arylalkyl groups include benzyl and phenethyl. The C₁-C₂₀ halogenated hydrocarbon groups may be those obtained by halogenating the above C₁-C₂₀ hydrocarbon groups. The hetero atom-containing groups include alkoxy groups represented by the formula: -OR; amino groups represented by the formula: -NR₂; or silyl groups represented by the formula: -SiR₃. In these formulae, R represents a C₁-C₂₀ hydrocarbon group which may be the same as exemplified above. Of these groups as R¹ to R³, preferred are a hydrogen atom and C₁-C₂₀ linear or branched alkyl groups.

R⁴ and R⁵ are each a hydrogen atom or a C₁-C₂₀ hydrocarbon group with the proviso that at least one of R⁴ and R⁵ represents a hydrogen atom, aryl or substituted aryl. When both of R⁴ and R⁵ are hydrogen atoms, α-olefins produced contain a less amount of by-products such as heavy components and waxes. The C₁-C₂₀ hydrocarbon groups may be the same as exemplified above. Examples of the aryl groups include phenyl, 1-naphjthyl and 2-naphthyl. Examples of the substituted aryl groups include those obtained by substituting at least one hydrogen atom of the above aryl groups with a hydrocarbon group or a halogen atom. Specific examples of the substituted aryl groups include linear or branched alkyl-substituted aryl groups such as 2-methylphenyl, 3-methylphenyl, 4-methylphenyl, 2-ethylphenyl, 3-ethylphenyl, 4-ethylphenyl, 2-n-propylphenyl, 3-n-propylphenyl, 4-n-propylphenyl, 2-isopropylphenyl, 3-isopropylphenyl, 4-isopropylphenyl, 2-isobutylphenyl, 3-isobutylphenyl, 4-isobutylphenyl, 2-tert-butylphenyl, 3-tert-butylphenyl, 4-tert-butylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 3,5-di-tert-butylphenyl and 2,4,6-trimethylphenyl; aromatic group-substituted aryl groups such as 2-phenylphenyl, 3-phenylphenyl and 4-phenylphenyl; and halogen-substituted aryl groups such as 2-florophenyl, 3-florophenyl, 4-florophenyl, 2-chlorophenyl, 3-chlorophenyl, 4-chlorophenyl, 2-bromophenyl, 3-bromophenyl, 4-bromophenyl, 2-iodophenyl, 3-iodophenyl, 4-iodophenyl, 2,3-difluorophenyl, 2,4-difluorophenyl, 2,5-difluorophenyl, 3,4-difluorophenyl, 3,5-difluorophenyl, 2,3,4,5,6-pentafluorophenyl and 3,5-bis(trifluoromethyl)phenyl.

R⁶ to R¹⁵ independently represent a hydrogen atom, a halogen atom, a C₁-C₂₀ hydrocarbon group or a hetero atom-containing group with the proviso that at least one of R⁶ and R¹⁰ or at least one of R¹¹ and R¹⁵ is a hydrogen atom, and R⁶ to R¹⁵ may be bonded to each other to form a ring. Preferably, at least one of R⁶ and R¹⁰ is a hydrogen atom, and at least one of R¹¹ and R¹⁵ is a hydrogen atom. When this requirement is met, α-olefins produced have a less amount of by-products such as heavy components and waxes. The halogen atoms, the C₁-C₂₀ hydrocarbon groups and the hetero atom-containing groups may be the same as exemplified above. As to the R⁶ to R¹⁵, preferred are a hydrogen atom and C₁-C₂₀ aliphatic hydrocarbon groups.

X represents a covalent or ionic bonding group, and when a plurality of X groups are present, these groups may be the same or different. Specific examples of X include a hydrogen atom, a halogen atom, a C₁-C₂₀ (preferably C₁-C₁₀) hydrocarbon group, C₁-C₂₀ (preferably C₁-C₁₀) alkoxy, amino, a C₁-C₂₀ (preferably C₁-C₁₂) phosphorus-containing group (e.g., diphenylphosphino), a C₁-C₂₀ (preferably C₁-C₁₂) silicon-containing group (e.g., trimethylsilyl or trimethylsilylmethyl), and halogen-containing boron anion (e.g., BF₄-). Of these groups, preferred are a halogen atom and C₁-C₂₀ hydrocarbon groups. The halogen atoms include chlorine, bromine and iodine. Of these halogen atoms, chlorine is preferred.

The suffix n is a valence of M. Specifically, n is a number of 0 to 3, preferably 2.

In the general formula (1), the preferred combinations of the substituent groups are as follows.

R⁴ and R⁵ both are hydrogen atoms; at least one of R⁶ and R¹⁰ is a hydrogen atom; and at least one of R¹¹ and R¹⁵ is a hydrogen atom.

R⁴ and R⁵ are phenyl, methyl or a hydrogen atom with the proviso that at least one of R⁴ and R⁵ is phenyl; and/or R¹ to R³ are all hydrogen atoms; and/or R⁶ to R¹⁵ are independently a hydrogen atom or alkyl, preferably methyl, ethyl, propyl or isopropyl, more preferably methyl or ethyl; and/or X is a monovalent anion, preferably a monovalent anion selected from a halogen atom and alkyl.

The above combinations are especially preferably used for producing α-olefins with a less amount of by-products such as heavy components and waxes.

Specific examples of the transition metal compounds represented by the above general formula (1) include the following compounds 1 to 48 and 63 to 65.

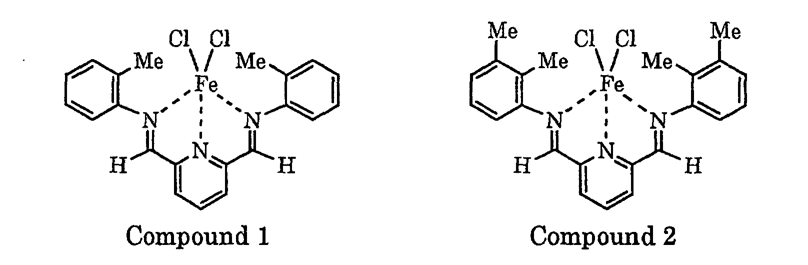

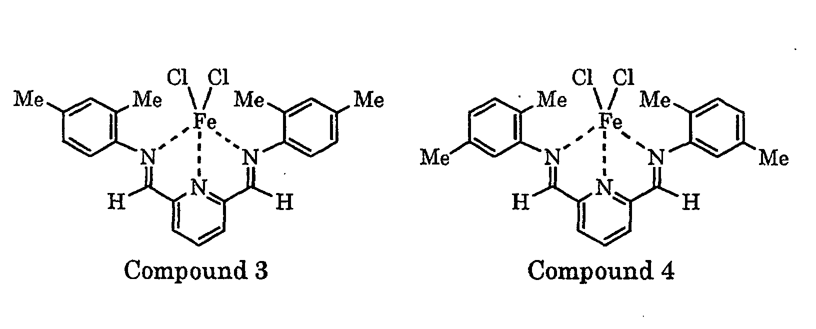

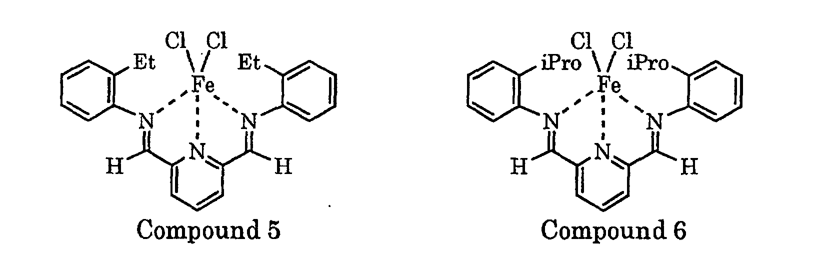

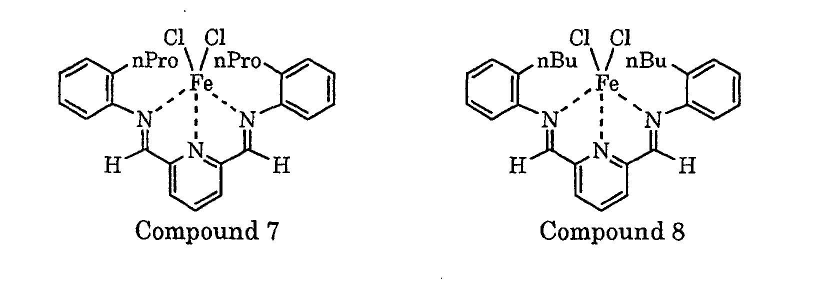

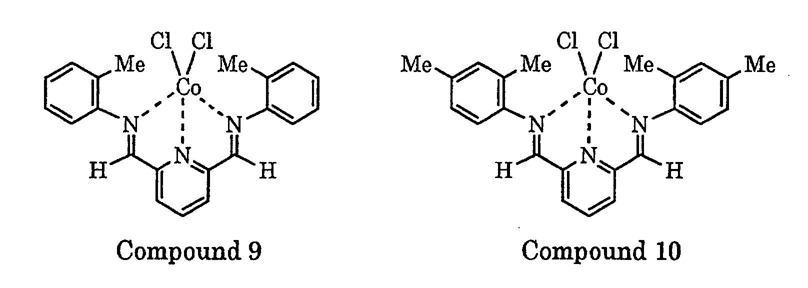

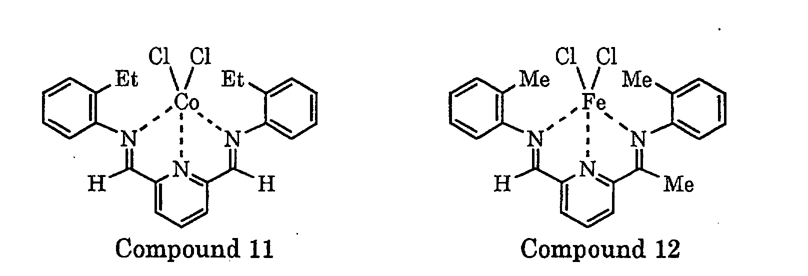

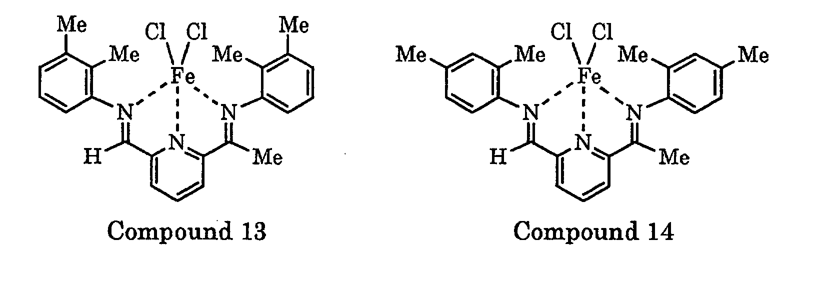

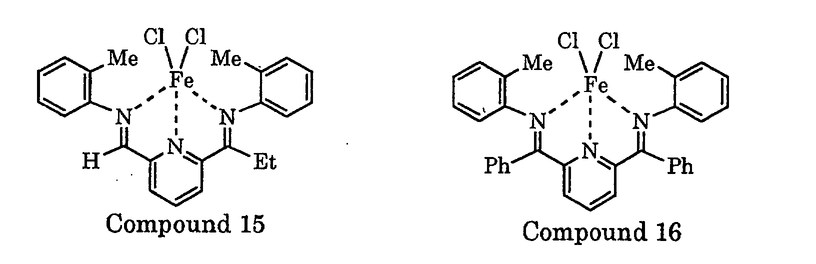

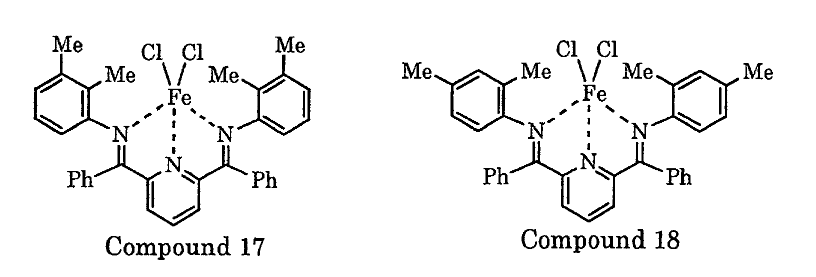

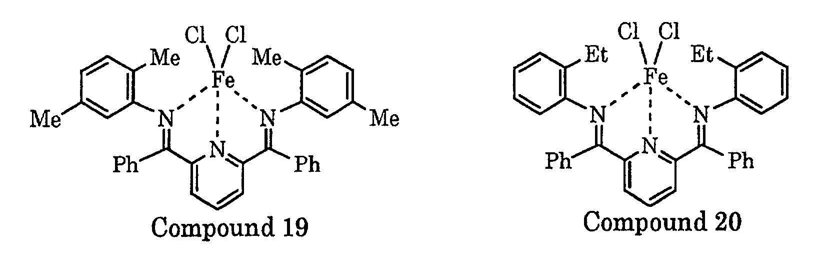

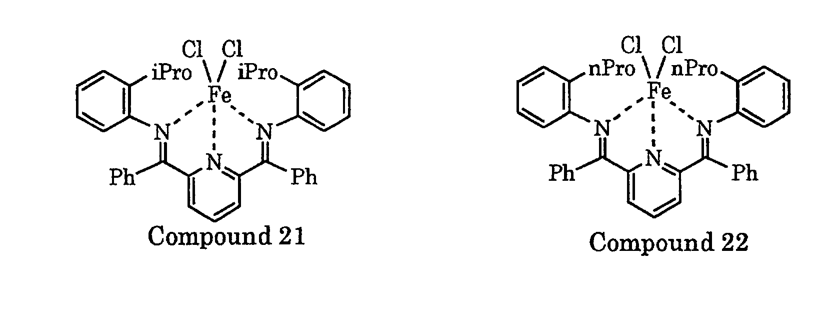

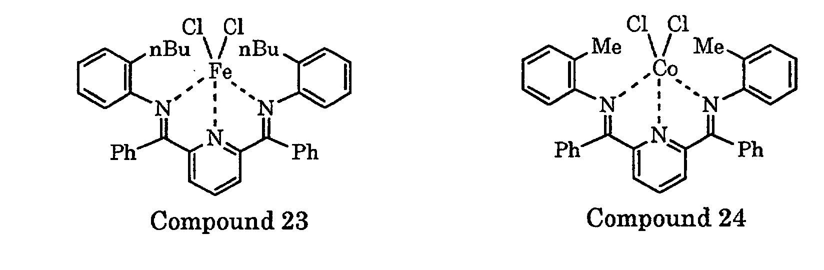

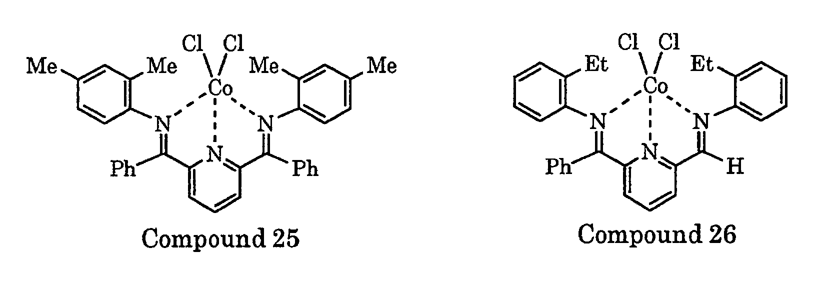

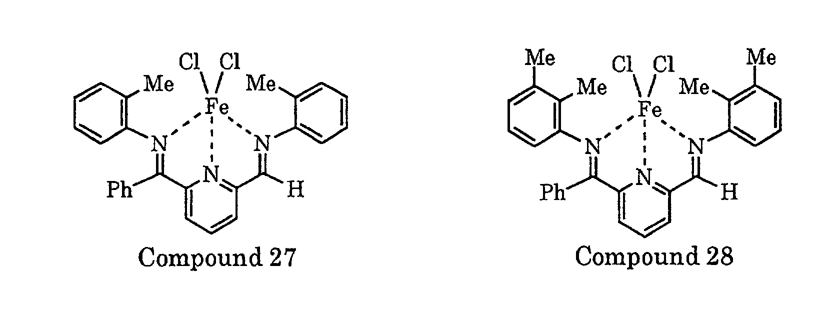

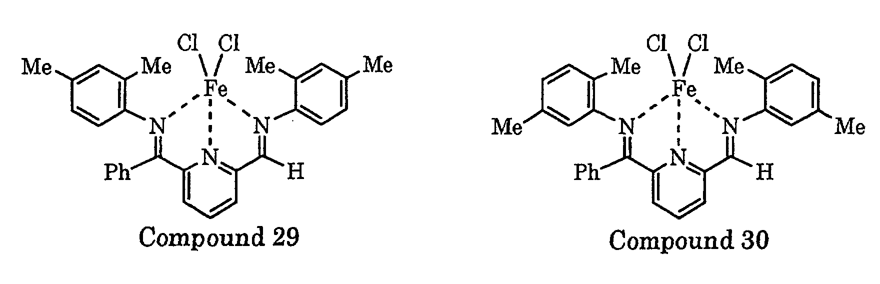

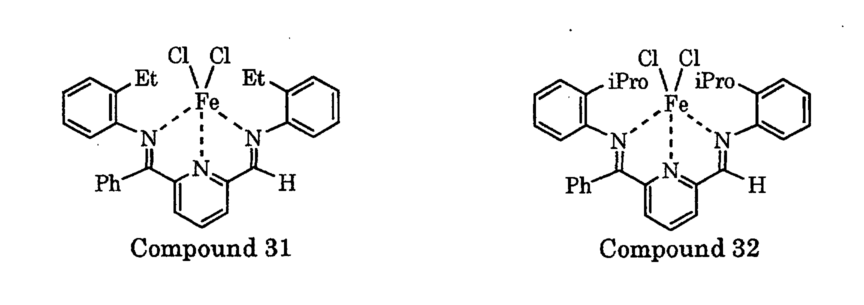

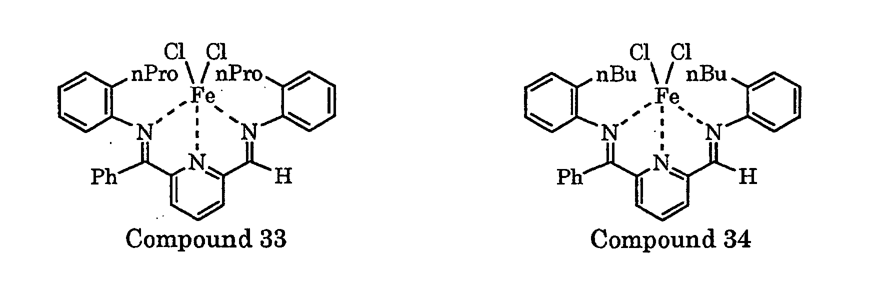

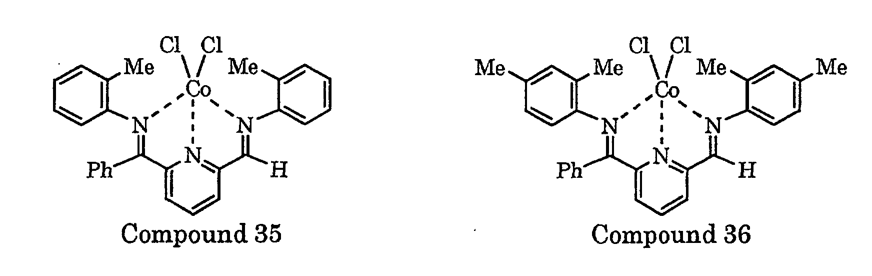

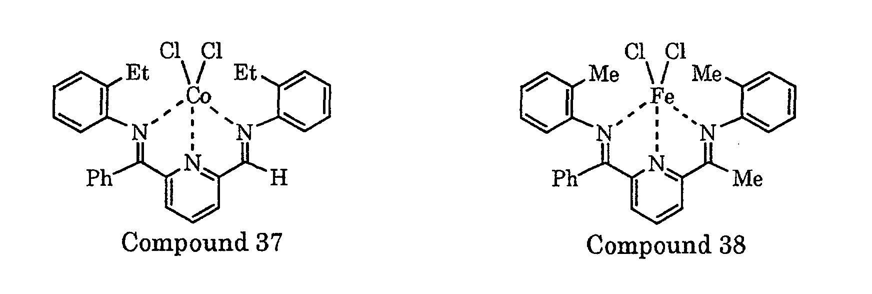

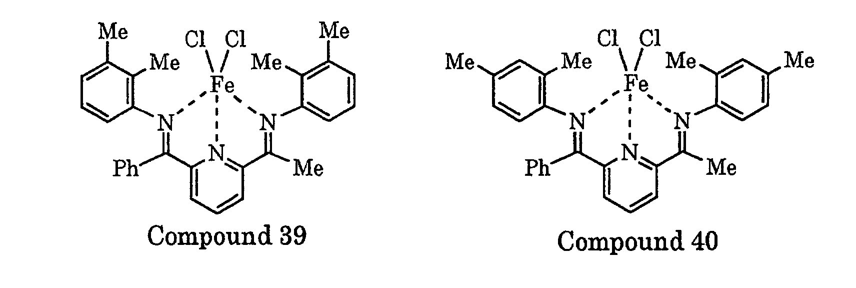

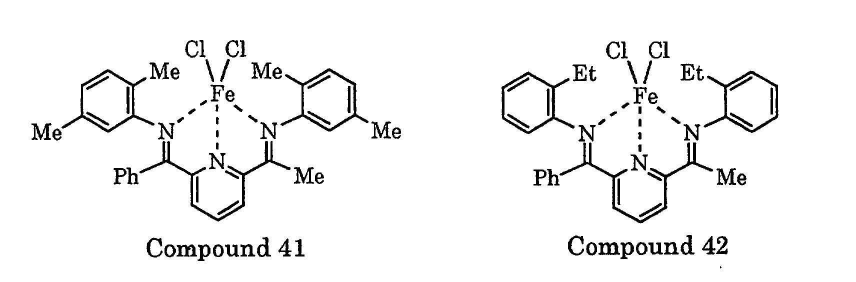

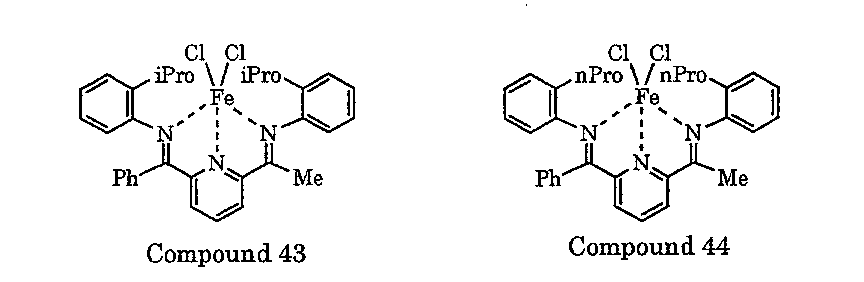

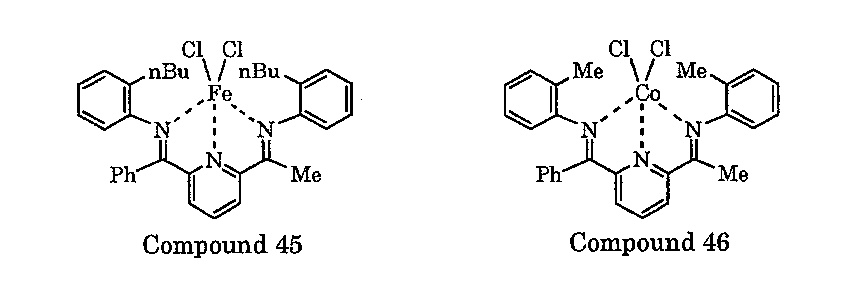

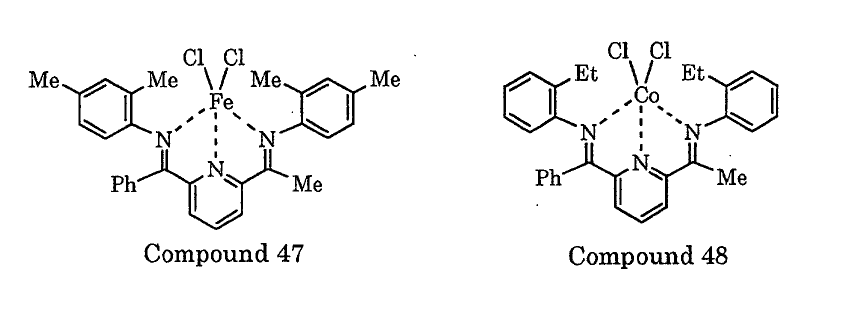

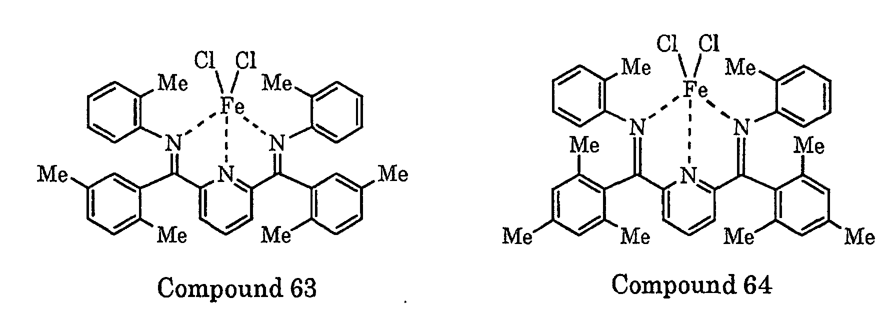

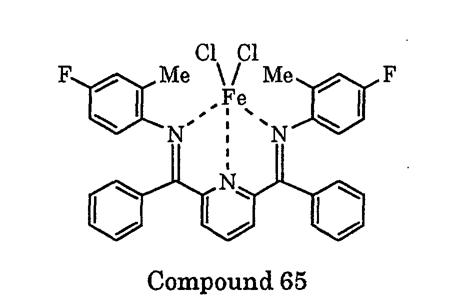

Of these compounds, preferred are the compounds 1, 2, 3, 4, 16, 17, 18, 19, 63, 64 and 65.

An example of the process for producing the transition metal compound represented by the general formula (1) will be described below.

The ligand (diimine compound) of the transition metal compound according to the present invention may be produced by reacting a ketone compound represented by the general formula (3) with an aniline compound represented by the general formula (4).

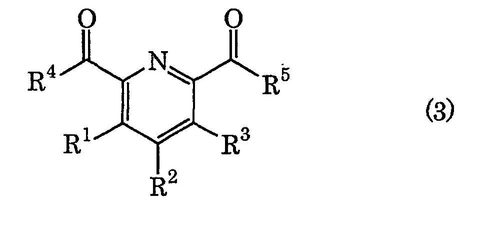

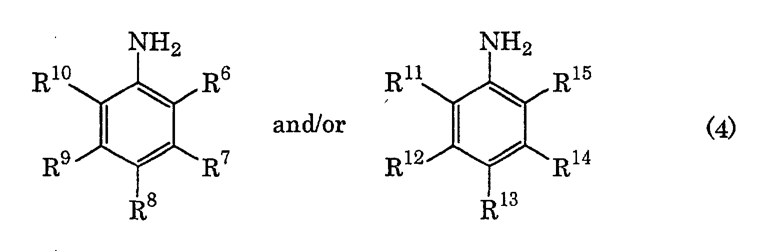

The reaction between these compounds may be carried out by using an organic acid such as formic acid as a catalyst. Further, the transition metal compound represented by the general formula (1) may be produced by reacting the thus obtained ligand (diimine compound) with a halide of the transition metal M (MX¹ₙ wherein X¹ is halogen) or an hydrate thereof.

The second transition metal compound of the present invention is represented by the general formula (2): 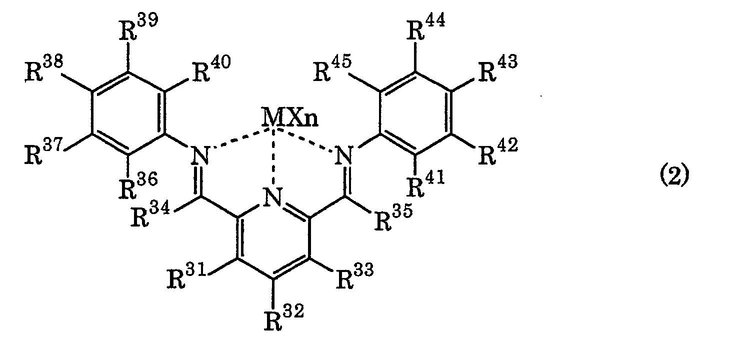 wherein M is a transition metal selected from the group consisting of elements of Groups 8 to 10 of the Periodic Table; R³¹ to R³³ are independently a hydrogen atom, a halogen atom, a C₁-C₂₀ hydrocarbon group, a C₁-C₂₀ halogenated hydrocarbon group or a hetero atom-containing group; R³⁴ and R³⁵ are independently a hydrogen atom or a C₁-C₂₀ hydrocarbon group; R⁴⁰ and R⁴⁵ are each a C₁-C₂₀ hydrocarbon group; R³⁶ and R⁴¹ are each a hydrogen atom; R³⁷ to R³⁹ are independently a hydrogen atom, a halogen atom or a C₁-C₂₀ hydrocarbon group with the proviso that at least one of R³⁷ to R³⁹ represents a halogen atom; R⁴² to R⁴⁴ are independently a hydrogen atom, a halogen atom or a C₁-C₂₀ hydrocarbon group with the proviso that at least one of R⁴² to R⁴⁴ represents a halogen atom; R³¹ to R³⁵ or R³⁶ to R⁴⁵ may be bonded to each other to form a ring; X is a covalent or ionic bonding group, and when a plurality of X groups are present, these groups may be the same or different; and n is a valence of M.

In the general formula (2), M represents a transition metal selected from the group consisting of elements of Groups 8 to 10 of the Periodic Table. Of these transition metals, preferred are iron and cobalt.

R³¹ to R³³ independently represent a hydrogen atom, a halogen atom, a C₁-C₂₀ hydrocarbon group, a C₁-C₂₀ halogenated hydrocarbon group or a hetero atom-containing group. The halogen atoms include fluorine, chlorine, bromine and iodine. The C₁-C₂₀ hydrocarbon groups include, for example, C₁-C₂₀ linear or branched alkyl, C₃-C₂₀ cycloalkyl, C₆-C₂₀ aryl and C₇-C₂₀ arylalkyl. Specific examples of the C₁-C₂₀ linear or branched alkyl groups include methyl, ethyl, n-propyl, isopropyl, n-butyl, isobutyl, sec-butyl, tert-butyl, various pentyl groups, various hexyl groups, various octyl groups, various decyl groups, various tetradecyl groups, various hexadecyl groups and various octadecyl groups. Specific examples of the C₃-C₂₀ cycloalkyl groups include cyclopentyl, cyclohexyl and cyclooctyl wherein an appropriate substitutent(s) such as lower alkyl may be bonded to the cycloalkyl ring. Specific examples of the C₆-C₂₀ aryl groups include phenyl, tolyl, xylyl, naphthyl and methylnaphthyl. Specific examples of the C₇-C₂₀ arylalkyl groups include benzyl and phenethyl. The C₁-C₂₀ halogenated hydrocarbon groups may be those formed by halogenating the above C₁-C₂₀ hydrocarbon groups. The hetero atom-containing groups include alkoxy groups represented by the formula: -OR; amino groups represented by the formula: -NR₂; and silyl groups represented by the formula: -SiR₃ wherein R represents a C₁-C₂₀ hydrocarbon group which may be the same as exemplified above. Of these groups for R³¹ to R³³, a hydrogen atom and C₁-C₂₀ linear or branched alkyl groups are preferred.

R³⁴ and R³⁵ independently represent a hydrogen atom or a C₁-C₂₀ hydrocarbon group. The C₁-C₂₀ hydrocarbon groups may be the same as exemplified above. Of these groups, preferred are C₁-C₂₀ linear hydrocarbon groups, and more preferred is methyl.

R⁴⁰ and R⁴⁵ are each a C₁-C₂₀ hydrocarbon group, and R³⁶ and R⁴¹ are each a hydrogen atom. The C₁-C₂₀ hydrocarbon groups may be the same as described above. Of these groups, preferred are C₁-C₂₀ linear hydrocarbon groups, and more preferred is methyl.

R³⁷ to R³⁹ independently represent a hydrogen atom, a halogen atom or a C₁-C₂₀ hydrocarbon group with the proviso that at least one of R³⁷ to R³⁹ represents a halogen atom. The halogen atoms and the C₁-C₂₀ hydrocarbon groups may be the same as exemplified above. Of these, preferred are fluorine as the halogen and C₁-C₂₀ linear hydrocarbon groups as the C₁-C₂₀ hydrocarbon groups.

R⁴² to R⁴⁴ independently represent a hydrogen atom, a halogen atom or a C₁-C₂₀ hydrocarbon group with the proviso that at least one of R⁴² to R⁴⁴ represents a halogen atom. The halogen atoms and the C₁-C₂₀ hydrocarbon groups may be the same as exemplified above. Of these, preferred are fluorine as the halogen and C₁-C₂₀ linear hydrocarbon groups as the C₁-C₂₀ hydrocarbon groups.

R³¹ to R³⁵ or R³⁶ to R⁴⁵ may be bonded to each other to form a ring.

X represents a covalent or ionic bonding group, and when a plurality of X groups are present, these groups may be the same or different. Specific examples of X include a hydrogen atom, a halogen atom, a C₁-C₂₀ (preferably C₁-C₁₀) hydrocarbon group, C₁-C₂₀ (preferably C₁-C₁₀) alkoxy, amino, a C₁-C₂₀ (preferably C₁-C₁₂) phosphorus-containing group (e.g., diphenylphosphino), a C₁-C₂₀ (preferably C₁-C₁₂) silicon-containing group (e.g., trimethylsilyl or trimethylsilylmethyl) or halogen-containing boron anion (e.g., BF₄-). Of these groups, preferred are halogen atoms and C₁-C₂₀ hydrocarbon groups. The halogen atoms include fluorine, chlorine, bromine and iodine. Of these halogen atoms, chlorine is preferred. The suffix n is a valence of M. Specifically, n is a number of 0 to 3, preferably 2.

In the general formula (2), the preferred combinations of the substituent groups are as follows.
R³⁴ and R³⁵ are each methyl or hydrogen; and/or
R³¹, R³², R³³, R³⁶ and R⁴¹ are all hydrogen atoms; and/or
R⁴⁰ and R⁴⁵ are each alkyl (preferably methyl, ethyl or propyl, more preferably methyl), at least one of R³⁷ to R³⁹ is fluorine (especially, R³⁷ is fluorine) and the remainders are each independently hydrogen, halogen or alkyl (preferably methyl, ethyl or propyl, more preferably methyl); and/or
at least one of R⁴² to R⁴⁴ is fluorine (especially, R⁴² is fluorine) and the remainders are each independently hydrogen, halogen or alkyl (preferably methyl, ethyl or propyl, more preferably methyl); and/or
X is a monovalent anion, preferably a monovalent anion selected from halogen and alkyl.

More preferred combinations of the substituent groups are:
R³⁴ and R³⁵ are each methyl or hydrogen; and/or
R³¹, R³², R³³, R³⁶ and R⁴¹ are all hydrogen atoms; and/or
R⁴⁰ and R⁴⁵ are each alkyl (preferably methyl, ethyl or propyl, more preferably methyl), at least one of R³⁷ to R³⁹ is fluorine (especially, R³⁷ is fluorine) and the remainders are each hydrogen; and/or
at least one of R⁴² to R⁴⁴ is fluorine (especially, R⁴² is fluorine) and the remainders are each hydrogen; and/or
X is a monovalent anion, preferably a monovalent anion selected from halogen and alkyl.

Specific examples of the transition metal compounds represented by the above general formula (2) include the following compounds 49 to 60.

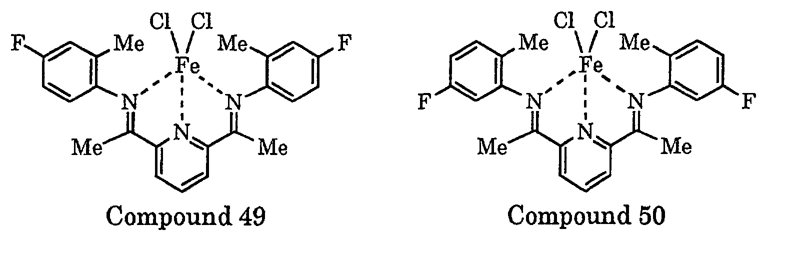

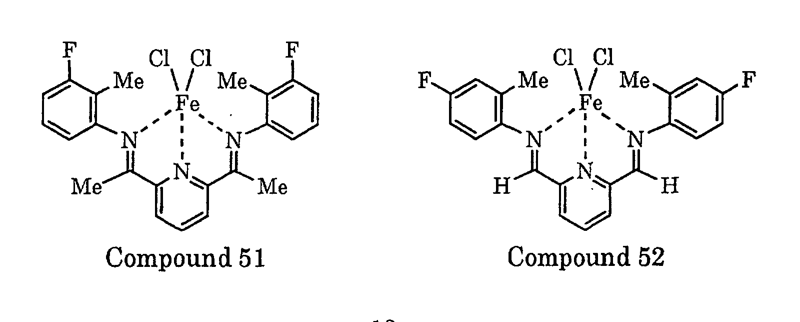

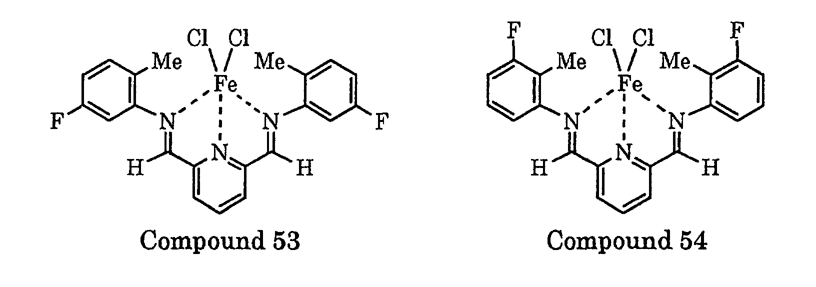

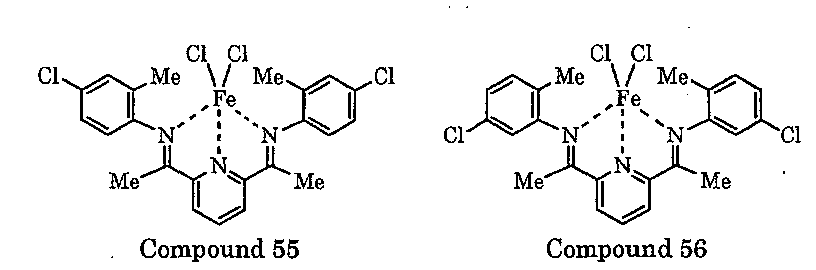

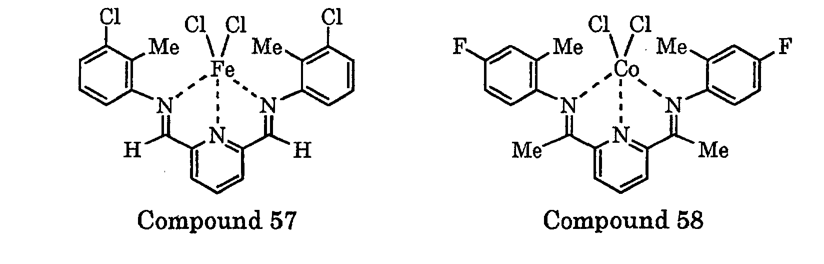

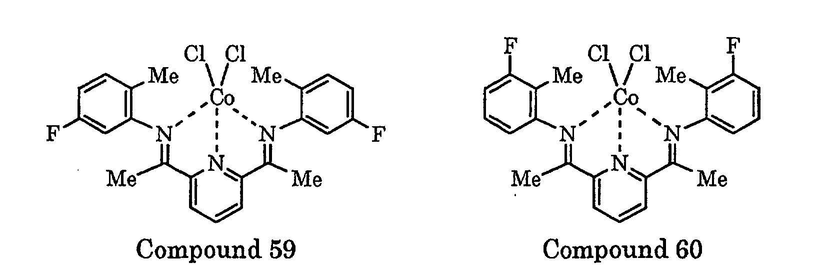

Of these compounds, the compounds 49, 50 and 51 are preferred, and the compound 50 is more preferred.

The transition metal compound represented by the general formula (2) may be produced by the same method as used for the production of the transition metal compound represented by the general formula (1).

### [2] Catalyst for production of α-olefins

The catalyst for the production of α-olefins according to the present invention comprises the following components (A) and (B).
(A) A transition metal compound containing a transition metal selected from the group consisting of elements of Groups 8 to 10 of the Periodic Table, which is represented by the general formula (1) or (2); and
(B) At least one compound selected from the group consisting of an organoaluminum compound (B-1), an ionic compound (B-2) capable of converting the transition metal compound into a cationic transition metal compound, a Lewis acid (B-3), and clay, clay mineral and an ion-exchangeable layer compound (B-4).

Also, the catalyst for the production of α-olefins according to the present invention comprises the following components (A), (B) and (C).
(A) A transition metal compound containing a transition metal selected from the group consisting of elements of Groups 8 to 10 of the Periodic Table, which is represented by the general formula (1) or (2);
(B) At least one compound selected from the group consisting of an organoaluminum compound (B-1), an ionic compound (B-2) capable of converting the transition metal compound into a cationic transition metal compound, a Lewis acid (B-3), and clay, clay mineral and an ion-exchangeable layer compound (B-4); and
(C) An organometallic compound.

The respective components of the catalyst are explained below.

### Component (A)

The component (A) is a transition metal compound containing a transition metal selected from the group consisting of elements of Groups 8 to 10 of the Periodic Table, which is represented by the general formula (1) or (2).

### Component (B)

The component (B) is at least one compound selected from the group consisting of an organoaluminum compound (B-1), an ionic compound (B-2) capable of converting the transition metal compound into a cationic transition metal compound, a Lewis acid (B-3), and clay, clay mineral and an ion-exchangeable layer compound (B-4).

### (B-1) Organoaluminum compound

The organoaluminum compounds include alkyl-containing aluminum compounds represented by the following general formula (5).

R¹⁶ ₘAl(OR¹⁶)ₙX² ₃₋ₘ₋ₙ (5)

wherein R¹⁶ is C₁-C₈, preferably C₁-C₄ alkyl, and when two or more R¹⁶ groups are present, these groups may be the same or different; X² is hydrogen or halogen; and m is 0<m≤ 3, preferably 2 or 3, more preferably 3, and n is 0≤n<3, preferably 0 or 1 with the proviso that 0<m+n≤3.

Examples of the organoaluminum compounds include linear aluminoxanes represented by the following general formula (6): 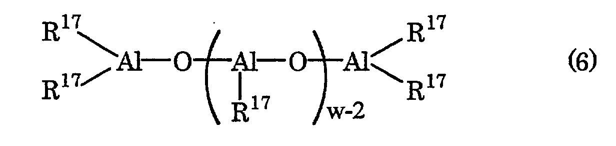 wherein R¹⁷ is a C₁-C₂₀, preferably C₁-C₁₂ hydrocarbon group such as alkyl, alkenyl, aryl and arylalkyl, or a halogen atom; w represents an average degree of polymerization, usually an integer of 2 to 50, preferably 2 to 40; and the groups of R¹⁷ may be the same or different, and cyclic aluminoxanes represented by the following general formula (7): 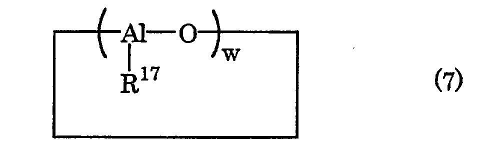 wherein R¹⁷ and w are the same as defined in the formula (6).

The above aluminoxanes may be produced by contacting alkyl aluminum with a condensing agent such as water. The contact method is not particularly restricted and may be conducted by any known manners. For example, there may be used (1) a method of dissolving an organoaluminum compound in an organic solvent, and then contacting the thus obtained solution with water; (2) a method of adding an organoaluminum compound at an initial stage of the polymerization, and then adding water at a later stage of the polymerization; (3) a method of reacting crystal water contained in metal salts or water adsorbed in inorganic or organic substances with an organoaluminum compound; and (4) a method of reacting tetraalkyl dialuminoxane with trialkylaluminum, and then reacting the reaction product with water.

The aluminoxanes may be either insoluble or soluble in hydrocarbon solvents. Preferred aluminoxanes are those soluble in hydrocarbon solvents and containing residual organoaluminum compounds in an amount of 10% by weight or less, preferably 3 to 5% by weight, more preferably 2 to 4% by weight when measured by ¹H-NMR. When the content of the residual organoaluminum compounds is more than 10% by weight, the catalytic activity tends to be deteriorated.

The preferred aluminoxanes may be produced, for example, by evaporating an aluminoxane-containing solution to dryness by heating under reduced pressure (called "dry-up method").

The components insoluble in hydrocarbon solvents may be removed from the aluminoxane, for example, by precipitating the insoluble components by gravity, and then separating the precipitated insoluble components by decantation. Alternatively, the precipitated insoluble components may be removed by centrifugal separation or the like. Preferably, the recovered soluble components may be further treated by passing through a G5 glass filter, etc., under a nitrogen stream to sufficiently remove the insoluble components therefrom. The content of gels in the thus obtained aluminoxanes increases with the passage of time. Therefore, the aluminoxanes are preferably used within 48 hours after the preparation thereof, more preferably immediately after the preparation. The ratio of the aluminoxane to the hydrocarbon solvent is not particularly restricted, but is preferably adjusted such that the amount of aluminum atom contained in the aluminoxane is 0.5 to 10 moles per one liter of the hydrocarbon solvent.

Examples of the hydrocarbon solvents include aromatic hydrocarbons such as benzene, toluene, xylene, cumene and cimene; aliphatic hydrocarbons such as pentane, hexane, heptane, octane, decane, dodecane, hexadecane and octadecane; alicyclic hydrocarbons such as cyclopentane, cyclohexane, cyclooctane and methylcyclopentane; and petroleum fractions such as naphtha, kerosene and light gas oil.

Examples of the preferred aluminoxanes include alkylaluminoxanes such as methylaluminoxane, ethylaluminoxane and isobutylaluminoxane. Of these aluminoxanes, more preferred are methylaluminoxane and tetraisobutyldialuminoxane. These aluminoxanes may be used alone or in the form of a mixture of two or more. Further, there may also be used such an aluminoxanes containing two or more different alkyl groups (e.g., methyl and isobutyl).

### (B-2) Ionic compound

The ionic compound capable of converting the transition metal compound into a cationic transition metal compound may be any suitable compounds. From the standpoint of efficiently forming active sites for the polymerization, the especially preferred ionic compounds may include those represented by the following general formulae (8) and (9):

([L¹-R¹⁸]^{h+})ₐ([Z]⁻)_{b} (8)

([L²]^{h+})ₐ([Z]⁻)_{b} (9)

wherein L² is M¹, R¹⁹R²⁰M², R²¹₃C or R²²M².

In the above general formulae (8) and (9), L¹ represents a Lewis base; [Z]⁻ represents a non-coordinating anion [Z¹]⁻ or [Z²]⁻ wherein [Z¹]⁻ is an anion containing an element and a plurality of groups bonded to the element, i.e., [M³G¹G²···G^{f}] wherein M³ is an element selected from the group consisting of elements of Groups 5 to 15, preferably Groups 13 to 15; G¹ to G^{f} are respectively hydrogen, halogen, C₁-C₂₀ alkyl, C₂-C₄₀ dialkylamino, C₁-C₂₀ alkoxy, C₆-C₂₀ aryl, C₆-C₂₀ aryloxy, C₇-C₄₀ alkylaryl, C₇-C₄₀ arylalkyl, C₁-C₂₀ halogenated hydrocarbon group, C₁-C₂₀ acyloxy, organometalloid group or C₂-C₂₀ hetero atom-containing hydrocarbon group, and at least two of G¹ to G^{f} may be bonded to each other to form a ring; f is an integer of [(valence of M³) + 1]; [Z²]- is a conjugated base composed of a Brφnsted acid solely or a combination of a Brφnsted acid and a Lewis acid, the Brφnsted acid showing a logarithm of reciprocal of acid dissociation constant (pKa) of -10 or lower, or a conjugated base generally defined as superstrong acids, and may be coordinated with a Lewis base; R¹⁸ is hydrogen, C₁-C₂₀ alkyl or C₆-C₂₀ aryl, alkylaryl or arylalkyl; R¹⁹ and R²⁰ are respectively cyclopentadienyl, substituted cyclopentadienyl, indenyl or fluorenyl; R²¹ is C₁-C₂₀ alkyl, aryl, alkylaryl or arylalkyl; R²² is a macrocyclic ligand such as tetraphenyl porphyrin and phthalocyanine; h represents an ionic valence of [L¹-R¹⁸] or [L²], i.e., an integer of 1 to 3; a is an integer of 1 or more; b is equal to h x a; M¹ represents an element selected from the group consisting of elements of Groups 1 to 3, 11 to 13 and 17 of the Periodic Table; and M² represents an element selected from the group consisting of elements of Groups 7 to 12 of the Periodic Table.

Specific examples of L¹ include amines such as ammonia, methylamine, aniline, dimethylamine, diethylamine, N-methylaniline, diphenylamine, N,N-dimethylaniline, trimethylamine, triethylamine, tri-n-butylamine, methyldiphenylamine, pyridine, p-bromo-N,N-dimethylaniline and p-nitro-N,N-dimethylaniline; phosphines such as triethylphosphine, triphenylphosphine and diphenylphosphine; thioethers such as tetrahydrothiophene; esters such as ethyl benzoate; and nitriles such as acetonitrile and benzonitrile.

Specific examples of R¹⁸ include hydrogen, methyl, ethyl, benzyl, trityl or the like. Specific examples of R¹⁹ and R²⁰ include cyclopentadienyl, methylcyclopentadienyl, ethylcyclopentadienyl, pentamethylcyclopentadienyl or the like. Specific examples of R²¹ include phenyl, p-tolyl, p-methoxyphenyl or the like. Specific examples of R²² include tetraphenylporphyrin, phthalocyanine, allyl, methallyl or the like. Specific examples of M¹ include Li, Na, K, Ag, Cu, Br, I,I₃ or the like. Specific examples of M² include Mn, Fe, Co, Ni, Zn or the like.

In [Z¹]-, i.e., [M³G¹G²···G^{f}], specific examples of M³ include B, Al, Si, P, As, Sb or the like. Of these, preferred are B and Al. Specific examples of G¹ to G^{f} include dialkylamino groups such as dimethylamino and diethylamino; alkoxy or aryloxy groups such as methoxy, ethoxy, n-butoxy and phenoxy; hydrocarbon groups such as methyl, ethyl, n-propyl, isopropyl, n-butyl, isobutyl, n-octyl, n-eicosyl, phenyl, p-tolyl, benzyl, 4-t-butylphenyl and 3,5-dimethylphenyl; halogen atoms such as fluorine, chlorine, bromine and iodine; hetero atom-containing hydrocarbon groups such as p-fluorophenyl, 3,5-difluorophenyl, pentachlorophenyl, 3,4,5-trifluorophenyl, pentafluorophenyl, 3,5-bis(trifluoromethyl)phenyl and bis(trimethylsilyl)methyl; and organometalloid groups such as pentamethylantimony, trimethylsilyl, trimethylgermyl, diphenylarsine, dicyclohexylantimony and diphenylboron.

Specific examples of the non-coordinating anion, i.e., the conjugated base [Z²]- composed of a Brφnsted acid alone or a combination of a Brφnsted acid and a Lewis acid, the Brφnsted acid having a pKa of -10 or lower, include trifluoromethanesulfonic acid anion (CF₃SO₃)⁻, bis(trifluoromethanesulfonyl)methyl anion, bis(trifluoromethanesulfonyl)benzyl anion, bis(trifluoromethanesulfonyl)amide, perchloric acid anion (ClO₄)-, trifluoroacetic acid anion (CF₃CO₂)-, hexafluoroantimony anion (SbF₆) ⁻, fluorosulfonic acid anion (FSO₃)-, chlorosulfonic acid anion (ClSO₃)-, fluorosulfonic acid anion/antimony pentafluoride [(FSO₃/SbF₅)-], fluorosulfonic acid anion/arsenic pentafluoride [(FSO₃/AsF₅)-], trifluoromethanesulfonic acid anion/antimony pentafluoride [(CF₃SO₃/SbF₅)-] or the like.

Specific examples of compounds as the (B-2) components include triethylammonium tetraphenylborate, tri-n-butylammonium tetraphenylborate, trimethylammonium tetraphenylborate, tetraethylammonium tetraphenylborate, methyl(tri-n-butyl)ammonium tetraphenylborate, benzyl(tri-n-butyl)ammonium tetraphenylborate, dimethyldiphenylammonium tetraphenylborate, triphenyl(methyl) ammonium tetraphenylborate, trimethylanilinium tetraphenylborate, methylpyridinium tetraphenylborate, benzylpyridinium tetraphenylborate, methyl(2-cyanopyridinium) tetraphenylborate, triethylammonium tetrakis(pentafluorophenyl)borate, tri-n-butylammonium tetrakis(pentafluorophenyl)borate, triphenylammonium tetrakis(pentafluorophenyl)borate, tetra-n-butylammonium tetrakis(pentafluorophenyl)borate, tetraethylammonium tetrakis(pentafluorophenyl)borate, benzyl(tri-n-butyl)ammonium tetrakis(pentafluorophenyl)borate, methyldiphenylammonium tetrakis(pentafluorophenyl)borate, triphenyl(methyl)ammonium tetrakis(pentafluorophenyl)borate, methylanilinium tetrakis(pentafluorophenyl)borate, dimethylanilinium tetrakis(pentafluorophenyl)borate, trimethylanilinium tetrakis(pentafluorophenyl)borate, methylpyridinium tetrakis(pentafluorophenyl)borate, benzylpyridinium tetrakis(pentafluorophenyl)borate, methyl(2-cyanopyridinium) tetrakis(pentafluorophenyl)borate, benzyl(2-cyanopyridinium) tetrakis(pentafluorophenyl)borate, methyl(4-cyanopyridinium) tetrakis(pentafluorophenyl)borate, triphenylphosphonium tetrakis(pentafluorophenyl)borate, dimethylanilinium tetrakis[bis(3,5-ditrifluoromethyl)phenyl]borate, ferrocenium tetraphenylborate, silver tetraphenylborate, trityl tetraphenylborate, tetraphenylporphyrin manganese tetraphenylborate, ferrocenium tetrakis(pentafluorophenyl)borate, (1,1'-dimethylferrocenium) tetrakis(pentafluorophenyl)borate, decamethylferrocenium tetrakis(pentafluorophenyl)borate, silver tetrakis(pentafluorophenyl)borate, trityl tetrakis(pentafluorophenyl)borate, lithium tetrakis(pentafluorophenyl)borate, sodium tetrakis(pentafluorophenyl)borate, tetraphenylporphyrin manganese tetrakis(pentafluorophenyl)borate, silver tetrafluoroborate, silver hexafluorophosphate, silver hexafluoroarsenate, silver perchlorate, silver trifluoroacetate, silver trifluoromethanesulfonate or the like. Of these (B-2) components, preferred are boron compounds.

### (B-3) Lewis acid

The Lewis acids used in the present invention are not particularly restricted, and may be in the from of either organic compounds or solid inorganic compounds. The preferred organic compounds as Lewis acids are organoboron compounds or organoaluminum compounds, and the preferred inorganic compounds as Lewis acids are magnesium compounds or aluminum compounds in view of efficient formation of active sites. The organoaluminum compounds include, for example, (2,6-di-t-butyl-4-methylphenoxy)aluminummethyl and (1,1-bi-2-naphthoxy)aluminummethyl. The magnesium compounds include, for example, magnesium chloride and diethoxy magnesium. The aluminum compounds include, for example, aluminum oxide and aluminum chloride. The organoboron compounds include, for example, triphenylboron, tris(pentafluorophenyl)boron, tris[3,5-bis(trifluoromethyl)phenyl]boron, tris[(4-fluoromethyl)phenyl]boron, trimethylboron, triethylboron, tri-n-butylboron, tris(fluoromethyl)boron, tris(pentafluoroethyl)boron, tris(nonafluorobutyl)boron, tris(2,4,6-trifluorophenyl)boron, tris(3,5-difluorophenyl)boron, tris[3,5-bis(trifluoromethyl)phenyl]boron, bis(pentafluorophenyl)fluoroboron, diphenylfluoroboron, bis(pentafluorophenyl)chloroboron, dimethylfluoroboron, diethylfluoroboron, di-n-butylfluoroboron, pentafluorophenyldifluoroboron, phenyldifluoroboron, pentafluorophenyldichloroboron, methyldifluoroboron, ethyldifluoroboron and n-butyldifluoroboron. These Lewis acids may be used alone or in the form of a mixture of any two or more thereof.

### (B-4) Clay, clay mineral and ion-exchangeable layer compound

The clays are substances in the form of aggregates of fine hydrated silicate minerals, exhibit a plasticity when kneaded with an appropriate amount of water and a rigidity when dried, and are sintered together when baked at a high temperature. The clay minerals are the hydrated silicates as a main component of the clays. In the preparation of the catalyst for the production of α-olefins, either clays or clay minerals may be used. The clays and clay minerals may be either natural or synthesized ones.

The ion-exchangeable layer compounds are those compounds having such a crystal structure that layers constituted by ionic bonds are weakly bonded together so as to be stacked one over another in parallel, and containing exchangeable ions therein. Some of the clay minerals are present in the form of such an ion-exchangeable layer compound.

The clay minerals include, for example, phyllosilicate minerals such as phyllosilicic acid and phyllosilicates. Examples of natural phyllosilicates include smectite group such as montmorillonite, saponite and hectorite; mica group such as illite and sericite; and a mixed layered minerals of smectite group and mica group, or mica group and vermiculite group. Examples of synthetic phyllosilicates include Tetrasilicon Fluoride Mica, Laponite, Smectone or the like. In addition, there may also be used non-clay, ionic crystalline compounds having a layer crystal structure, such as α-Zr(HPO₄)₂, γ-Zr(HPO₄)₂, α-Ti(HPO₄)₂ and γ-Ti(HPO₄)₂.

Also, clays and clay minerals which are not involved in the ion-exchangeable layer compounds, include clays called "bentonite" due to a low content of montmorillonite, Kibushi clay orgairome clay having a high content of montmorillonite and other components, fibrous sepiolite or palygorskite, and amorphous or low-crystalline allophene and imogolite.

Of these (B-4) components, preferred are clays and clay minerals, specifically phyllosilicates; more preferred are smectites; most preferred is montmorillonite.

The clays, clay minerals and ion-exchangeable layer compounds used as the (B-4) component have a volume-average particle size of preferably 10 µm or smaller, more preferably 3 µm or smaller. Further, the clays, clay minerals and ion-exchangeable layer compounds used as the (B-4) component preferably have such a particle size distribution that the volume-average particle size thereof is 10 µm or smaller and the content of particles having a volume-average particle size of 3 µm or smaller is 10% by weight or less, more preferably have such a particle size distribution that the volume-average particle size thereof is 10 µm or smaller and the content of particles having a volume-average particle size of 1.5 µm or smaller is 10% by weight or less. Here, the volume-average particle size and the content of the specific particles are measured, for example, using a particle size analyzer such as "CIS-1" available from GALAI Production Ltd., which is capable of determining a particle size from the measurement of laser beam transmittance of particles.

Furthermore, the clays, clay minerals and ion-exchangeable layer compounds used as the (B-4) component are preferably subjected to chemical treatments to remove impurities therefrom and modify structure or properties thereof, thereby transforming these substances into a more preferred form as a catalyst component.

For example, the clays, clay minerals and ion-exchangeable layer compounds may be treated with an organosilane compound represented by the following general formula (10):

R²³ ₙSiX³ ₄₋ₙ (10)

wherein R²³ is hydrogen or a substituent having carbon atom or silicon atom which is directly bonded to Si; X³ is halogen or a substituent having oxygen atom or nitrogen atom which is directly bonded to Si; when a plurality of R²³ or X³ groups are present, these groups may be the same or different; and n is an integer of 1 to 3.

The organosilane compounds may be bis-silyl compounds, polynuclear polysiloxane or polynuclear polysilazane represented by the following general formula (11):

X³ ₄₋ₙSi(CH₂)ₘSiX³ ₄₋ₙ (11)

wherein X³ is halogen or a substituent having oxygen atom or nitrogen atom directly bonded to Si; when a plurality of X³ groups are present, these X³ groups may be the same or different; m is 1 to 10; and n is 1 to 3.

Specific examples of the organosilane compounds represented by the above general formulae, include trialkylsilyl chlorides such as trimethylsilyl chloride, triethylsilyl chloride, triisopropylsilyl chloride, t-butyldimethylsilyl chloride, t-butyldiphenylsilyl chloride and phenethyldimethylsilyl chloride; dialkylsilyl dichlorides such as dimethylsilyl dichloride, diethylsilyl dichloride, diisopropylsilyl dichloride, di-n-hexylsilyl dichloride, dicyclohexylsilyl dichloride, docosylmethylsilyl dichloride, bis(phenethyl)silyl dichloride, methylphenethylsilyl dichloride, diphenylsilyl dichloride, dimethylsilyl dichloride and ditolylsilyl dichloride; diarylsilyl dichlorides; and alkylarylsilyl dichlorides.

In addition, as the organosilane compounds, there may also be used silyl halides formed by substituting the chloride moieties of the above-exemplified organosilane compounds with another halogen atom; disilazanes such as bis(trimethylsilyl)amide, bis(triethylsilyl)amide, bis(triisopropylsilyl)amide, bis(dimethylethylsilyl)amide, bis(diethylmethylsilyl)amide, bis(dimethylphenylsilyl)amide, bis(dimethyltolylsilyl)amide and bis(dimethylmenthylsilyl)amide; trialkylsilyl hydroxides such as trimethylsilyl hydroxide, triethylsilyl hydroxide, triisopropylsilyl hydroxide, tertbutyldimethylsilyl hydroxide and phenethyldimethylsilyl hydroxide; polysilanols generally called peralkylpolysiloxypolyols; bis-silyls such as bis(methyldichlorosilyl)methane, 1,2-bis(methyldichlorosilyl)ethane, bis(methyldichlorosilyl)octane and bis(triethoxysilyl)ethane; and hydride-containing silanes such as dimethylchlorosilane, (N,N-dimethylamino)dimethylsilane and diisobutylchlorosilane. These organosilane compounds may be used alone or in the form of a mixture of any two or more thereof.

Of these organosilane compounds, preferred are those containing at least one alkyl group directly bonded to the silicon atom, such as alkylsilyl halides, especially dialkylsilyl halides. The clays, clay minerals and ion-exchangeable layer compounds may be effectively treated with these organosilane compounds in the presence of water. In this case, water serves for breaking the crystal structure (especially, laminated structure) of the clay to thereby enhance the efficiency of contact between the organosilane compound and the clay. That is, water acts for spreading the distance between crystal layers of the clay, thereby accelerating the diffusion of the organosilane compounds into the laminated crystal structure. More specifically, the organosilane-treated (B-4) component may be prepared in the following manner. First, water is added to the (B-4) component to form an aqueous colloidal solution of the (B-4) component. Then, the organosilane compounds are added to the thus obtained aqueous colloidal solution of the (B-4) component, and the resultant mixture is stirred while heating to treat the (B-4) component with the organosilane compounds. The treatment is conducted at a temperature ranging from ordinary temperature to 200°C, and preferably at a temperature near 100°C for facilitating the preparation process. The treating time varies depending upon kind of (B-4) component and treating temperature used, and may be in the range of 0.5 to 24 hours.

The amount of the organosilane compound used in the above contact treatment of the (B-4) component is 0.001 to 1,000 moles, preferably 0.01 to 100 moles in terms of silicon atom contained in the organosilane compounds, based on one kilogram of the (B-4) component.

These (B-4) components may be used alone or in the from of a mixture of any two or more thereof.

In the polymerization catalyst of the present invention, the molar ratio of the catalyst component (A) to the catalyst component (B) is preferably 1:1 to 1:1,000,000, more preferably 1:10 to 1:10,000 when the (B-1) compound is used as the (B) component; preferably 10:1 to 1:100, more preferably 2:1 to 1:10 when the (B-2) compound is used as the (B) component; and preferably 10:1 to 1:200, more preferably 5:1 to 1:100, still more preferably 2:1 to 1:50 when the (B-3) compound is used as the (B) component. Further, when the (B-4) compound is used as the (B) compound, the amount of the (A) component used is usually 0.01 to 100 millimoles, preferably 0.1 to 1 millimole, based on an unit weight (g) of the (B-4) compound. The (B-4) compound may be preferably pretreated with the organometallic compound (C) described hereinafter, especially with the organoaluminum compound. In this case, the amount of the organometallic compound (C) used for the pretreatment is usually 0.1 to 1,000 millimoles, preferably 1 to 100 millimoles based on an unit weight (g) of the (B-4) compound. However, even when the organometallic compound (C) is used in an excessive amount relative to the (B-4) compound, the excess part thereof may be removed out of the reaction system by washing a suspension or slurry of the clay or the like with an appropriate solvent.

These (B-1), (B-2), (B-3) and (B-4) compounds may be respectively used alone or in the form of a mixture of any two or more thereof.

### (C) Organometallic compound

The catalyst for the production of α-olefins according to the present invention may further contain the organometallic compound (C), if required.

As the organometallic compounds as the (C) component, organoaluminum compounds are preferred in view of low cost and easy availability, though organozinc compounds or organomagnesium compounds may also be used. Specific examples of the organoaluminum compounds include trialkylaluminums such as trimethylaluminum, triethylaluminum, tripropylaluminum, triisobutylaluminum and tri-tert-butylaluminum; halogen- or alkoxy-containing alkyl aluminums such as dimethylaluminum chloride, diethylaluminum chloride, dimethylaluminum methoxide and diethylaluminum ethoxide; and alumoxanes such as methylalumoxane, ethylalumoxane and isobutylalumoxane. Of these organoaluminum compounds, preferred are trialkylaluminums.

The molar ratio of the catalyst component (C) to the catalyst component (A) is preferably 10:1 to 1:10,000, more preferably 1:1 to 1:2,000, most preferably 1:10 to 1:1,000.

The contact between the (A), (B) and (C) components may be preferably conducted in an inert gas stream such as argon and nitrogen and in the presence of a hydrocarbon solvent such as pentane, hexane, heptane, toluene and xylene. Further, the reaction system is preferably free from water or active hydrogen-containing compounds such as those containing hydroxyl or amino groups, since these substances are harmful to the catalyst. For this reason, the reaction system may be preliminarily treated with the (C) component to remove water or the active hydrogen-containing compounds therefrom. That is, the use of the catalyst prepared by preliminarily contacting the (A), (B) and (C) components with each other is preferred. Meanwhile, it is not necessarily required that the (C) component is used in the preparation of the catalyst. The (C) component may be added to the reaction system for the production of α-olefins. The contact between the respective components may be conducted at a temperature between ordinary temperature and the boiling point of solvent used.

### [3] Process for the production of α-olefins

The process for producing α-olefins according to the present invention will be described below. In the process for the production of α-olefins according to the present invention, ethylene is oligomerized using the above catalyst and , if required, in the presence of the (C) component. The oligomerization reaction may be conducted by any methods without particular limitations, for example, by a solution polymerization method using an appropriate solvent, a non-solvent, liquid-phase reaction method using substantially no solvent, or a gas-phase reaction method, and may also be conducted by either a continuous method or a batch method. Examples of the solvents used include hydrocarbon solvents such as pentane, hexane, heptane, cyclohexane, benzene and toluene. These solvents may be used alone or in the form of a mixture of any two or more thereof. When the solvent is used, the amount of the catalyst used is preferably adjusted such that the amount of the (A) component contained therein is usually 0.1 to 100 micromoles (µmol), preferably 1 to 20 micromoles per one liter of the solvent, in view of reaction activity.

The reaction conditions are not particularly restricted. The reaction temperature is usually in the range of -78 to 20°C, preferably from ordinary temperature to 150°C. The ethylene pressure in the reaction system is usually in the range of from ordinary pressure to 15 MPa, preferably from ordinary pressure to 8 MPa, more preferably from ordinary pressure to 5 MPa. The molecular weight of the oligomer prepared upon the oligomerization may be controlled by any known methods, for example, by appropriately selecting reaction conditions such as temperature and pressure.

The present invention will be described in more detail with reference to the following examples, but these examples are not intended to limit the present invention thereto.

First, the method for the analysis of the obtained α-olefins is described.

The α-olefins were analyzed by gas chromatography to determine the composition and purity thereof. The analysis conditions are shown below.

### (1) Composition analysis

The measurement was conducted using a gas chromatograph GC-14A manufactured by Shimadzu Co., Ltd., together with an FID detector. The column used was "TC-1" manufactured by G.L. Science Co., Ltd. (length: 15 m, inner diameter: 0.53 mm, membrane thickness: 1.5 µm). The carrier gas used was He. The temperature program was set so that the temperature was maintained at 40°C for 5 minutes, and then increased to 320°C at a temperature rise rate of 10°C per minute, and that temperature was maintained for 10 minutes. Both the injection and the detector were maintained at 320°C. As described in Examples hereinafter, undecane was used as an internal standard for the measurement.

### (2) Purity analysis

The measurement was conducted using a gas chromatograph GC-14A manufactured by Shimadzu Co., Ltd., together with an FID detector. The column used was "Ultra-2" manufactured by Hewlett Packard Corp. (length: 25 m, inner diameter: 0.20 mm, membrane thickness: 0.33 µm). The carrier gas and make-up gas used were He and nitrogen, respectively. The temperature program was set so that the temperature was increased from 90°C to 200°C at a temperature rise rate of 1.5°C per minute, and, immediately after reaching 200°C, further increased to 270°C at a temperature rise rate of 8°C per minute, and then that temperature was maintained for 70 minutes. Both the injection and the detector were maintained at 270°C.

A fraction having a carbon number of x is represented by Cx, and the purity of Cx means the content (% by weight) of normal-1-olefin contained in the fraction having a carbon number of x.

### EXAMPLE 1

### Preparation of [2,6-pyridinedicarboxyaldehyde-bis(2-methylphenylimine)] iron dichloride complex (Compound 1)

### (1) Preparation of ligand: Preparation of 2,6-pyridinedicarboxyaldehyde-bis(2-methylphenylimine)

Into a 300-ml flask, 50 ml of ethanol, 680 mg of 2,6-pyridinedicarboxyaldehyde (molecular weight: 135.12; 5 mmol) and 2.14 g of 2-methylaniline (molecular weight: 107.16; 20 mmol) were charged and sufficiently stirred to form a homogeneous solution. To the obtained solution was added 0.3 ml of formic acid, and the resultant mixture was stirred for 12 hours to permit the reaction to proceed. The reaction solution was allowed to stand at -20°C for 24 hours, thereby obtaining light-yellow crystals. The crystals were filtered, washed with ethanol and then dried under reduced pressure. As a result of ¹H-NMR measurement, it was confirmed that the crystals were titled 2,6-pyridinedicarboxyaldehyde-bis(2-methylphenylimine) (molecular weight: 313.41; 500 mg; 1.6 mmol; yield: 32%).
¹H-NMR [90 MHz, solvent: CDCl₃, standard: tetramethylsilane (δ0.00)]: 62.42 (6H, CH₃, s), δ6.9-7.4 (8H, benzene ring), δ7.93 (1H, p-position of pyridine ring, dd), δ8.34 (2H, m-position of pyridine ring, d), δ8.59 (2H, imine-H, s).

### (2) Preparation of complex: Preparation of [2,6-pyridinedicarboxyaldehyde-bis(2-methylphenylimine)] iron dichloride complex (Compound 1)

Then, a tetrahydrofuran solution (30 ml) of the obtained 2,6-pyridinedicarboxyaldehyde-bis(2-methylphenylimine) (molecular weight: 313.41; 500 mg; 1.6 mmol) and a tetrahydrofuran solution (30 ml) of iron (II) chloride tetrahydrate (formula weight: 198.81; 278 mg; 1.4 mmol) were charged into a 100-ml Schlenk tube and stirred for 12 hours under a nitrogen stream to allow the reaction to proceed. The obtained yellowish green solids were filtered, washed with tetrahydrofuran and then dried under reduced pressure, thereby obtaining the title complex (Compound 1) (molecular weight: 440.16; 460 mg; 1.05 mmol; yield: 75%).

### EXAMPLE 2

### Preparation of [2,6-pyridinedicarboxyaldehyde-bis(2,3-dimethylphenylimine)] iron dichloride complex (Compound 2)

### (1) Preparation of ligand: Preparation of 2,6-pyridinedicarboxyaldehyde-bis(2,3-dimethylphenylimine)

Into a 300-ml flask, 50 ml of ethanol, 680 mg of 2,6-pyridine dicarboxyaldehyde (molecular weight: 135.12; 5 mmol) and 2.42 g of 2,3-dimethyl aniline (molecular weight: 121.18; 20 mmol) were charged and sufficiently stirred to form a homogeneous solution. To the obtained solution was added 0.3 ml of formic acid, and the resultant mixture was stirred for 12 hours to permit the reaction to proceed. The obtained light-yellow crystals were filtered, washed with ethanol and then dried under reduced pressure. As a result of ¹H-NMR measurement, it was confirmed that the crystals were titled 2,6-pyridinedicarboxyaldehyde-bis(2,3-dimethylphenylimine) (molecular weight: 341.47; 1.05 g; 3.07 mmol; yield: 62%).
¹H-NMR [90 MHz, solvent: CDCl₃, standard: tetramethylsilane (δ0.00)]: δ2.34 (6H, 2-CH₃, s), δ2.34 (6H, 3-CH₃, s), δ6.8-7.2 (6H, benzene ring), δ7.92 (1H, p-position of pyridine ring, dd), δ8.34 (2H, m-position of pyridine ring, d), δ8.57 (2H, imine-H, s).

### (2) Preparation of complex: Preparation of [2,6-pyridinedicarboxyaldehyde-bis(2,3-dimethylphenylimine)] iron dichloride complex (Compound 2)

Then, a tetrahydrofuran solution (30 ml) of the obtained 2,6-pyridinedicarboxyaldehyde-bis(2,3-dimethylphenylimine) (molecular weight: 341.47; 1.0 g; 2.93 mmol) and a tetrahydrofuran solution (30 ml) of iron (II) chloride tetrahydrate (formula weight: 198.81; 557 mg; 2.8 mmol) were charged into a 100-ml Schlenk tube and stirred for 12 hours under a nitrogen stream to allow the reaction to proceed. The obtained yellowish green solids were filtered, washed with tetrahydrofuran and then dried under reduced pressure, thereby obtaining the titled complex (Compound 2) (molecular weight: 468.22; 1.18 g; 2.52 mmol; yield: 90%).

### EXAMPLE 3

### Preparation of [2,6-pyridinedicarboxyaldehyde-bis(2,4-dimethylphenylimine)] iron dichloride complex (Compound 3)

### (1) Preparation of ligand: Preparation of 2,6-pyridinedicarboxyaldehyde-bis(2,4-dimethylphenylimine)

Into a 300-ml flask, 50 ml of ethanol, 680 mg of 2,6-pyridinedicarboxyaldehyde (molecular weight: 135.12; 5 mmol) and 2.42 g of 2,4-dimethyl aniline (molecular weight: 121.18; 20 mmol) were charged and sufficiently stirred to form a homogeneous solution. To the obtained solution was added 0.3 ml of formic acid, and the resultant mixture was stirred for 12 hours to allow the reaction to proceed. The obtained light-yellow crystals were filtered, washed with ethanol and then dried under reduced pressure. As a result of ¹H-NMR measurement, it was confirmed that the crystals were titled 2,6-pyridinedicarboxyaldehyde-bis(2,4-dimethylphenylimine) (molecular weight: 341.47; 0.67 g; 1.65 mmol; yield: 33%).
¹H-NMR [90 MHz, solvent: CDCl₃, standard: tetramethylsilane (δ0.00)]: δ2,35 (6H, 4-CH₃, s), δ2.40 (6H, 2-CH₃, s), δ6.9-7.2 (6H, benzene ring), δ7.92 (1H, p-position of pyridine ring, dd), δ8.32 (2H, m-position of pyridine ring, d), δ8.59 (2H, imine-H, s).

### (2) Preparation of complex: Preparation of [2,6-pyridinedicarboxyaldehyde-bis(2,4-dimethylphenylimine)] iron dichloride complex (Compound 3)

Then, a tetrahydrofuran solution (30 ml) of the above obtained 2,6-pyridinedicarboxyaldehyde-bis(2,4-dimethylphenylimine) (molecular weight: 341.47; 410 mg; 120 mmol) and a tetrahydrofuran solution (30 ml) of iron (II) chloride tetrahydrate (formula weight: 198.81; 199 mg; 1.0 mmol) were charged into a 100-ml Schlenk tube and stirred for 12 hours under a nitrogen stream to allow the reaction to proceed. The obtained yellowish green solids were filtered, washed with tetrahydrofuran and then dried under reduced pressure, thereby obtaining the titled complex (Compound 3) (molecular weight: 468.22; 440 mg; 0.94 mmol; yield: 94%).

### EXAMPLE 4

### Preparation of [2,6-pyridinedicarboxyaldehyde-bis(2,5-dimethylphenylimine)] iron dichloride complex (Compound 4)

### (1) Preparation of ligand: Preparation of 2,6-pyridinedicarboxyaldehyde-bis(2,5-dimethylphenylimine)

Into a 300-ml flask, 50 ml of methanol, 680 mg of 2,6-pyridine dicarboxyaldehyde (molecular weight: 135.12; 5 mmol) and 2.42 g of 2,4-dimethylaniline (molecular weight: 121.18; 20 mmol) were charged and sufficiently stirred to form a homogeneous solution. To the obtained solution was added 0.3 ml of formic acid, and the resultant mixture was stirred for 12 hours to allow the reaction to proceed. The obtained light-yellow crystals were filtered, washed with methanol and then dried under reduced pressure. As a result of ¹H-NMR measurement, it was confirmed that the crystals were titled 2,6-pyridinedicarboxyaldehyde-bis(2,5-dimethylphenylimine) (molecular weight: 341.47; 1.44 g; 4.22 mmol; yield: 84%).
¹H-NMR [90 MHz, solvent: CDCl₃, standard: tetramethylsilane (δ0.00)]: δ2.36 (6H, 2-CH₃, s), δ2.36 (6H, 5-CH₃, s), δ6.8-7.3 (6H, benzene ring), δ7.91 (1H, p-position of pyridine ring, dd), δ8.32 (2H, m-position of pyridine ring, d), δ8.58 (2H, imine-H, s).

### (2) Preparation of complex: Preparation of [2,6-pyridinedicarboxyaldehyde-bis(2,5-dimethylphenylimine)] iron dichloride complex (Compound 4)

Then, a tetrahydrofuran solution (30 ml) of the obtained 2,6-pyridinedicarboxyaldehyde-bis(2,5-dimethylphenylimine) (molecular weight: 341.47; 1.0 g; 2.93 mmol) and a tetrahydrofuran solution (30 ml) of iron (II) chloride tetrahydrate (formula weight: 198.81; 557 mg; 2.8 mmol) were charged into a 100-ml Schlenk tube and stirred for 12 hours under a nitrogen stream to allow the reaction to proceed. The obtained yellowish green solids were filtered, washed with tetrahydrofuran and then dried under reduced pressure, thereby obtaining the titled complex (Compound 4) (molecular weight: 468.22; 1.03 g; 2.2 mmol; yield: 79%).

### COMPARATIVE EXAMPLE 1

### Preparation of [2,6-diacetylpyridine-bis(2-methylphenylimine)] iron dichloride complex (Compound 61)

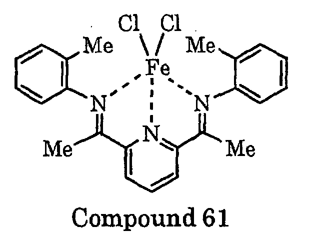

### (1) Preparation of ligand: Preparation of 2,6-diacetylpyridine-bis(2-methylphenylimine)

Into a 300-ml flask, 100 ml of ethanol, 1.63 g of 2,6-diacetylpyridine (molecular weight: 163.18; 10 mmol) and 4.29 g of 2-methyl aniline (molecular weight: 107.16; 40 mmol) were charged and sufficiently stirred to form a homogeneous solution. To the obtained solution was added 0.6 ml of formic acid, and the resultant mixture was stirred for 12 hours to allow the reaction to proceed. The reaction solution was allowed to stand at -78°C for one hour, thereby obtaining light-yellow crystals. The obtained light-yellow crystals were filtered, washed with methanol and then dried under reduced pressure. As a result of ¹H-NMR measurement, it was confirmed that the crystals were titled 2,6-diacetylpyridine-bis(2-methylphenylimine) (molecular weight: 341.47; 780 mg; 2.28 mmol; yield: 23%).
¹H-NMR [90 MHz, solvent: CDCl₃, standard: tetramethylsilane (δ0.00)]: δ2.17 (6H, 2-CH₃, s), δ2.40 (6H, imine-CH₃, s), δ6.5-7.3 (8H, benzene ring), δ7.90 (1H, p-position of pyridine ring, dd), δ8.42 (2H, m-position of pyridine ring, d).

### (2) Preparation of complex: Preparation of [2,6-diacetylpyridine-bis(2-methylphenylimine)] iron dichloride complex (Compound 61)

Then, a tetrahydrofuran solution (30 ml) of the obtained 2,6-diacetylpyridine-bis(2-methylphenylimine) (molecular weight: 341.47; 780 mg; 2.28 mmol) and a tetrahydrofuran solution (30 ml) of iron (II) chloride tetrahydrate (formula weight: 198.81; 400 mg; 2.0 mmol) were charged into a 100-ml Schlenk tube and stirred for 12 hours under a nitrogen stream to allow the reaction to proceed. The obtained bluish violet solids were filtered, washed with tetrahydrofuran and then dried under reduced pressure, thereby obtaining the titled complex (Compound 61) (molecular weight: 468.22; 930 mg; 2.0 mmol; yield: 100%).

### COMPARATIVE EXAMPLE 2

Preparation of [2,6-diacetylpyridine-bis(2,4-dimethylphenylimine)] iron dichloride complex (Compound 62)

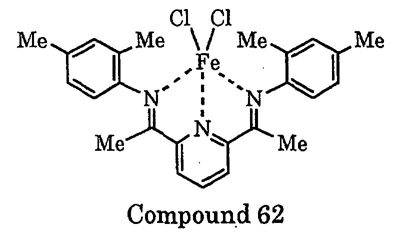

### (1) Preparation of ligand: Preparation of 2,6-diacetylpyridine-bis(2,4-dimethylphenylimine)

Into a 300-ml flask, 100 ml of methanol, 1.63 g of 2,6-diacetylpyridine (molecular weight: 163.18; 10 mmol) and 4.84 g of 2,4-dimethylaniline (molecular weight: 121.18; 40 mmol) were charged and sufficiently stirred to form a homogeneous solution. To the solution was added 0.6 ml of formic acid, and the resultant mixture was stirred for 12 hours to allow the reaction to proceed. The reaction solution was allowed to stand at -78°C for one hour, thereby obtaining light-yellow crystals which were then filtered, washed with methanol and then dried under reduced pressure. As a result of ¹H-NMR measurement, it was confirmed that the crystals were titled 2,6-diacetylpyridine-bis(2,4-dimethylphenylimine) (molecular weight: 369.52; 1.60 g; 4.33 mmol; yield: 43%).
¹H-NMR [90 MHz, solvent: CDCl₃, standard: tetramethylsilane (δ0.00)]: δ2.10 (6H, 2-CH₃, s), δ2.34 (6H, 4-CH₃, s), δ2.34 (6H, imine-CH₃, s), δ6.5-7.2 (6H, benzene ring), 67.87 (1H, p-position of pyridine ring, dd), δ8.40 (2H, m-position of pyridine ring, d)

### (2) Preparation of complex: Preparation of [2,6-diacetylpyridine-bis(2,4-dimethylphenylimine)] iron dichloride complex (Compound 62)

Then, a tetrahydrofuran solution (40 ml) of the obtained 2,6-diacetylpyridine-bis(2,4-dimethylphenylimine) (molecular weight: 369.52; 1.0 g; 2.71 mmol) and a tetrahydrofuran solution (40 ml) of iron (II) chloride tetrahydrate (formula weight: 198.81; 497 mg; 2.5 mmol) were charged into a 100-ml Schlenk tube and stirred for 12 hours under a nitrogen gas stream to allow the reaction to proceed. The obtained bluish violet solids were filtered, washed with tetrahydrofuran and then dried under reduced pressure, thereby obtaining the titled complex (Compound 62) (molecular weight: 496.27; 1.24 g; 2.5 mmol; yield: 100%).

### EXAMPLE 5: Oligomerization of Ethylene

Into a 1-liter autoclave, 250 ml of toluene and 1.0 ml of a toluene solution of polymethylalumoxane (available from Toso Akzo Co., Ltd., concentration: 1 mmol/ml) were charged and then 0.5 ml of a 1-µmol/ml toluene suspension of Compound 1 prepared in Example 1 was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction.

After completion of the reaction, the autoclave was depressurized, and the gaseous components were introduced into a wet flow meter where a total volume thereof was measured. Then, the gaseous components were analyzed by gas chromatography to determine composition and quantities thereof. The reaction solution was analyzed by gas chromatography using n-undecane as internal standard to determine the quantity of α-olefins. The solid components were separated by filtration, dried at 120°C for 12 hours, and then subjected to quantitative analysis. As a result, it was confirmed that the total amount of the reaction products was 39 g. The oligomerization activity per unit weight of iron metal was 2,770 kg/g-Fe·h. The results of measurement of the composition and purity of the reaction products are shown in Tables 1 and 2, respectively. In the Tables, Cx represents a fraction having a carbon number of x; C⁺₂₀ represents a fraction having a carbon number of 20 or more; and the "heavy components" represent solid polymers obtained by the polymerization.

### EXAMPLE 6

The same procedure as in Example 5 was repeated except that the reaction was conducted by using Compound 2 obtained in Example 2. The results are shown in Tables 1 and 2.

### EXAMPLE 7

The same procedure as in Example 5 was repeated except that the reaction was conducted by using Compound 3 obtained in Example 3. The results are shown in Tables 1 and 2.

### EXAMPLE 8

The same procedure as in Example 5 was repeated except that the reaction was conducted by using Compound 4 obtained in Example 4. The results are shown in Tables 1 and 2.

### COMPARATIVE EXAMPLE 3

The same procedure as in Example 5 was repeated except that the reaction was conducted by using Compound 61 obtained in Comparative Example 1. The results are shown in Tables 1 and 2.

### COMPARATIVE EXAMPLE 4

The same procedure as in Example 5 was repeated except that the reaction was conducted by using Compound 62 obtained in Comparative Example 2. The results are shown in Tables 1 and 2.

**Table 1**

| Composition of Products | | | | | | |
|---|---|---|---|---|---|---|
| | Examples | | | | Comparative Examples | |
| | 5 | 6 | 7 | 8 | 3 | 4 |
| Total weight of products | 39 | 15 | 12 | 8 | 116 | 72 |
| (g) | | | | | | |
| Activity (kg/g-Fe·hr) | 2,770 | 1,080 | 850 | 550 | 8,290 | 5,180 |
| Composition (wt%) | | | | | | |
| C₄ | 32.5 | 37.2 | 26.9 | 27.3 | 9.8 | 8.4 |
| C₆ | 24.5 | 25.5 | 22.2 | 22.0 | 10.2 | 9.2 |
| C₈ | 16.6 | 16.0 | 17.7 | 16.5 | 9.9 | 9.0 |
| C₁₀ | 10.0 | 8.5 | 10.0 | 10.0 | 8.0 | 8.0 |
| C₁₂ | 6.2 | 4.8 | 7.4 | 6.7 | 7.0 | 6.9 |
| C₁₄ | 3.7 | 2.7 | 5.0 | 4.4 | 5.9 | 6.0 |
| C₁₆ | 2.2 | 1.6 | 3,3 | 3.0 | 5.1 | 5.2 |
| C₁₈ | 1.3 | 1.0 | 2.2 | 2.1 | 4.4 | 4.5 |
| C₂₀⁺ | 2.7 | 2.6 | 5.3 | 5.7 | 20.3 | 22.0 |
| heavy components | 0.3 | 0.0 | 0.0 | 2.1 | 19.4 | 20.7 |

**Table 2**

| Purity of Products | | | | | | |
|---|---|---|---|---|---|---|
| | Examples | | | | Comparative Examples | |
| | 5 | 6 | 7 | 8 | 3 | 4 |
| Purity (%) | | | | | | |
| C₁₄ | 96.05 | 96.57 | 97.20 | 97.21 | 96.55 | 96.33 |
| C₁₆ | 94.76 | 95.62 | 96.67 | 96.19 | 95.62 | 95.16 |
| C₁₈ | 94.37 | 94.70 | 96.24 | 95.30 | 94.95 | 94.69 |

### EXAMPLE 9: Preparation of Compound 16

### (1) Preparation of ligand precursor: 2,6-dibenzoylpyridine

A round-bottom two-neck flask equipped with a reflux condenser was charged with 12.2 g (91.5 ml) of AlCl₃, and the inner atmosphere of the flask was replaced with nitrogen. To the flask was added a benzene solution comprising 50 ml of benzene containing 6.12 g (30.0 mmol) of 2,6-pyridinedicarbonyl dichloride, and the resultant mixture was heated while stirring and refluxed for 5 hours. After allowing the obtained reaction mixture to stand for cooling, an aqueous NaHCO₃ solution was gradually added to deactivate AlCl₃. The reaction solution was extracted with toluene. The organic phase was dried over anhydrous MgSO₄, and then the solvent was distilled off. The obtained reaction product was purified by silica gel column chromatography (hexane/ethyl acetate = 10/1 by volume) to obtain 6.78 g of 2,6-dibenzoylpyridine as a ligand precursor (yield: 80%).
¹H-NMR, [270 MHz, solvent: CDCl₃, standard: chloroform (δ7.24)]: δ7.42 (4H, m-position of benzene ring, dd), δ7.57 (2H, p-position of benzene ring, dd), δ8.07-8.17 (5H, o-position of benzene ring and 4-position of pyridine ring, m), δ8.28 (2H, 3-position of pyridine ring, d)

### (2) Preparation of ligand: 2,6-dibenzoylpyridine-di(2-methylphenyl)imine

A solution prepared by dissolving 0.50 g (4.7 mmol) of o-toluidine in 10 ml of tetrahydrofuran (THF) was treated with 1.5 ml (2.4 mmol) of 1.6-mol/liter n-BuLi at -78°C to form a Li salt of o-toluidine. The obtained reaction solution was heated to room temperature and then slowly dropped to a THF solution (10 ml) containing 0.20 g (0.67 mmol) of 2,6-dibenzoyl pyridine. After stirring the resultant mixture for 30 minutes, excessive Li salt was deactivated with methanol, and then the solvent was distilled off under reduced pressure. The reaction product was then mixed with distilled water and toluene, and the solvent was distilled off from the organic phase. Further, the product was heated to 100°C under reduced pressure (1 mmHg) to remove the excessive toluidine, to obtain 0.32 g of 2,6-dibenzoylpyridine-di(2-methylphenyl)imine as a ligand (yield: 50%).

### (3) Preparation of iron complex (Compound 16)

A methanol solution containing an excess amount of FeCl₂(H₂O)₄ was added to a dichloromethane solution of the above ligand. It was confirmed that the aimed complex was produced immediately after the addition, and the reaction solution was colored dark blue. After distilling off the solvents, the residue was extracted with dichloromethane to obtain the iron complex (Compound 16).

### EXAMPLE 10: Oligomerization of Ethylene

Into a 1-liter autoclave, 250 ml of toluene and 2.0 ml of a toluene solution of polymethylalumoxane (available from Toso Akzo Co., Ltd., concentration: 1 mmol/ml) were charged and then 0.5 ml of a 1-µmol/ml toluene suspension of Compound 16 was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction.

After completion of the reaction, the autoclave was depressurized, and the gaseous components were introduced into a wet flow meter where a total volume thereof was measured. Then, the gaseous components were analyzed by gas chromatography to determine composition and quantities thereof. The reaction solution was also analyzed by gas chromatography using n-undecane as internal standard to determine a quantity of α-olefins. The solid components were separated by filtration, dried at 120°C for 12 hours, and then subjected to quantitative analysis. As a result, it was confirmed that a total amount of the reaction product was 69 g. The oligomerization activity per unit weight of iron metal was 4,900 kg/g-Fe-hr. The results of measurement of the composition and purity of the reaction products are shown in Tables 3 and 4, respectively. In the Tables, Cx represents a fraction having a carbon number of x; C⁺₂₀ represents a fraction having a carbon number of 20 or more; and the "heavy components" represent solid polymers obtained by the polymerization.

### COMPARATIVE EXAMPLE 5

The same procedure as in Example 10 was repeated except that the reaction was conducted by using Compound 61. The results are shown in Tables 3 and 4.

### EXAMPLE 11: Oligomerization of Ethylene (Compound 16)

The same procedure as in Example 10 was repeated except that the reaction was conducted at 70°C instead of 50°C. The results of the analysis of α-olefin distribution and purity are shown in Tables 3 and 4.

### EXAMPLE 12: Oligomerization of Ethylene (Compound 16)

The same procedure as in Example 10 was repeated except that the toluene solution of polymethylalumoxane was added in an amount of 4.0 ml instead of 2.0 ml, and the reaction was conducted at a temperature of 90°C instead of 50°C. The results of the analysis of α-olefin distribution and purity are shown in Tables 3 and 4.

### EXAMPLE 13: Oligomerization of Ethylene (Compound 16)

The same procedure as in Example 10 was repeated except that the reaction was conducted under a pressure of 3.5 MPa instead of 1.0 MPa. The results of the analysis of α-olefin distribution and purity are shown in Tables 3 and 4.

### EXAMPLE 14: Preparation of Compound 63

### (1) Preparation of ligand precursor: 2,6-di(2,5-xyloyl)pyridine

The same procedure as in Example 9(1) was repeated except that the reaction was conducted by using a p-xylene solution instead of the benzene solution. As a result, 10.1 g of 2,6-di(2,5-xyloyl)pyridine as a ligand precursor were obtained (yield: 98%).
¹H-NMR [270 MHz, solvent: CDCl₃, standard: chloroform (δ7.24)]: δ2.16 (6H, CH₃, s), δ2.21 (6H, CH₃, s), δ6.69-7.20 (6H, benzene ring, m), δ8.09 (1H, 4-position of pyridine ring, t), δ8.26 (2H, 3-position of pyridine ring, d).

### (2) Preparation of ligand: 2,6-di(2,5-xyloyl)pyridine-di(2-methylphenyl)imine

Into a solution of 1.03 g (3.0 mmol) of 2,6-di(2,5-xyloyl)pyridine prepared in the step (1) dissolved in 20 ml of methanol, 1.6 ml of o-toluidine (15.0 mmol) and several droplets of formic acid were added to initiate the reaction. After stirring the reaction solution over night, the precipitated solids were filtered out and washed with 5 ml of methanol three times, thereby obtaining 2,6-di(2,5-xyloyl)pyridine-di(2-methylphenyl)imine as the titled ligand (yield: 30%).
¹H-NMR [270 MHz, solvent: CDCl₃, standard: chloroform (δ7.24)]: δ1.68 (6H, CH₃, s), δ2.08 (6H, CH₃, s), δ2.29 (6H, CH₃, s), δ6.32-7.11 (14H, benzene ring, m), δ7.95 (1H, 4-position of pyridine ring, t), δ8.42 (2H, 3-position of pyridine ring, d).

### (3) Preparation of iron complex (Compound 63)

The same procedure as in Example 9(3) was repeated except that the reaction was conducted by using 2,6-di(2,5-xyloyl)pyridine-di(2-methylphenyl)imine prepared in the step (2) instead of 2,6-dibenzoylpyridine-di(2-methylphenyl)imine. As a result, the titled iron complex (Compound 63) was produced nearly quantitatively.

### EXAMPLE 15: Oligomerization of Ethylene (Compound 63)

The same procedure as in Example 10 was repeated except that the reaction was conducted by using Compound 63 prepared in Example 14 instead of Compound 16 and using a toluene solution of polymethyl alumoxane 4.0 ml instead of 2.0 ml. The results of the analysis of α-olefin distribution and purity are shown in Tables 3 and 4.

### EXAMPLE 16: Oligomerization of Ethylene (Compound 63)

The same procedure as in Example 15 was repeated except that the reaction was conducted at 70°C instead of 50°C. The results of the analysis of α-olefin distribution and purity are shown in Tables 3 and 4.

### EXAMPLE 17: Preparation of Compound 64

### (1) Preparation of ligand precursor: 2,6-di(2,4,6-mesityloyl)pyridine

The same procedure as in Example 9(1) was repeated except that the reaction was conducted by using a p-xylene solution instead of the benzene solution. As a result, 7.1 g of 2,6-di(2,4,6-mesityloyl)pyridine as a ligand precursor were obtained (yield: 64%).
¹H-NMR [270 MHz, solvent: CDCl₃, standard: chloroform (δ7.24)]:
δ1.81 (6H, CH₃, s), δ1.98 (6H, CH₃, s), δ2.31 (6H, CH₃, s), δ6.62 (2H, benzene ring, s), 66.73 (2H, benzene ring, s), δ8.02-8.28 (3H, pyridine ring, m)

### (2) Preparation of ligand: 2,6-di(2,4,6-mesityloyl)pyridine-di(2-methylphenyl)imine

The same procedure as in Example 9(2) was repeated except that the reaction was conducted by using 2,6-di(2,4,6-mesityloyl)pyridine prepared in the step (1) instead of 2,6-dibenzoylpyridine. As a result, 2,6-di(2,4,6-mesityloyl)pyridine-di(2-methylphenyl)imine as a ligand was obtained (yield: 65%).

### (3) Preparation of iron complex (Compound 64)

The same procedure as in Example 9(3) was repeated except that the reaction was conducted by using 2,6-di(2,4,6-mesityloyl)pyridine-di(2-methylphenyl)imine prepared in the step (2) instead of 2,6-dibenzoylpyridine-di(2-methylphenyl)imine. As a result, the titled iron complex (Compound 64) was produced nearly quantitatively.

### EXAMPLE 18: Oligomerization of Ethylene (Compound 64)

The same procedure as in Example 15 was repeated except that the reaction was conducted by using Compound 64 prepared in Example 17 instead of Compound 63. The results of the analysis of α-olefin distribution and purity are shown in Tables 3 and 4.

### EXAMPLE 19: Oligomerization of Ethylene (Compound 64)

The same procedure as in Example 18 was repeated except that the reaction was conducted at 70°C instead of 50°C. The results of the analysis of α-olefin distribution and purity are shown in Tables 3 and 4.

### EXAMPLE 20: Oligomerization of Ethylene (Compound 64)

The same procedure as in Example 18 was repeated except that the reaction was conducted at 90°C for 20 minutes instead of at 50°C for 30 minutes. The results of the analysis of α-olefin distribution and purity are shown in Tables 3 and 4.

### EXAMPLE 21: Preparation of Compound 65

### (1) Preparation of ligand: 2,6-dibenzoylpyridine-di(2-methyl-4-fluorophenyl)imine

The same procedure as in Example 14(2) was repeated except that the reaction was conducted by using 0.86 g (3.0 mmol) of 2,6-dibenzoylpyridine prepared in Example 9(1) instead of 1.03 g (3.0 mmol) of 2,6-di(2,5-xyloyl)pyridine and using 1.7 ml (15.0 mmol) of 4-fluoro-2-methylaniline instead of o-toluidine. As a result, 2,6-dibenzoylpyridine-di(2-methyl-4-fluorophenyl)imine as the titled ligand was obtained (yield: 65%).

### (2) Preparation of iron complex (Compound 65)

The same procedure as in Example 9(3) was repeated except that the reaction was conducted by using 2,6-dibenzoylpyridine-di(2-methyl-4-fluorophenyl)imine prepared in the step (1) instead of 2,6-dibenzoylpyridine-di(2-methylphenyl)imine. As a result, the titled iron complex (Compound 65) was produced nearly quantitatively.

### EXAMPLE 22: Oligomerization of Ethylene (Compound 65)

The same procedure as in Example 15 was repeated except that the reaction was conducted by using Compound 65 prepared in Example 21 instead of Compound 64. The results of the analysis of α-olefin distribution and purity are shown in Tables 3 and 4.

### COMPARATIVE EXAMPLE 6: Oligomerization of Ethylene (Compound 61)

The same procedure as in Comparative Example 5 was repeated except that the reaction was conducted under a pressure of 3.5 MPa instead of 1.0 MPa. The results of the analysis of α-olefin distribution and purity are shown in Tables 3 and 4.

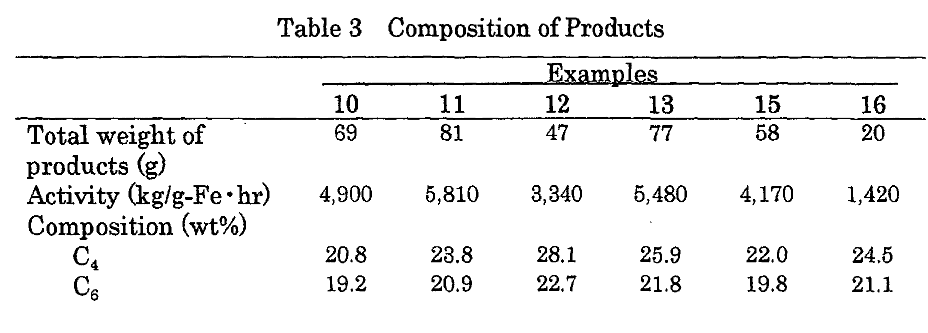

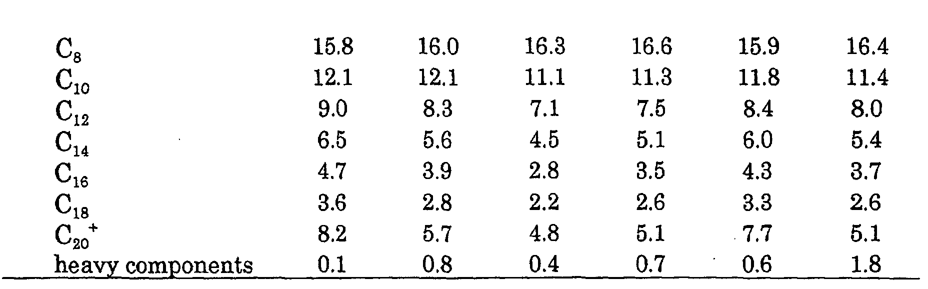

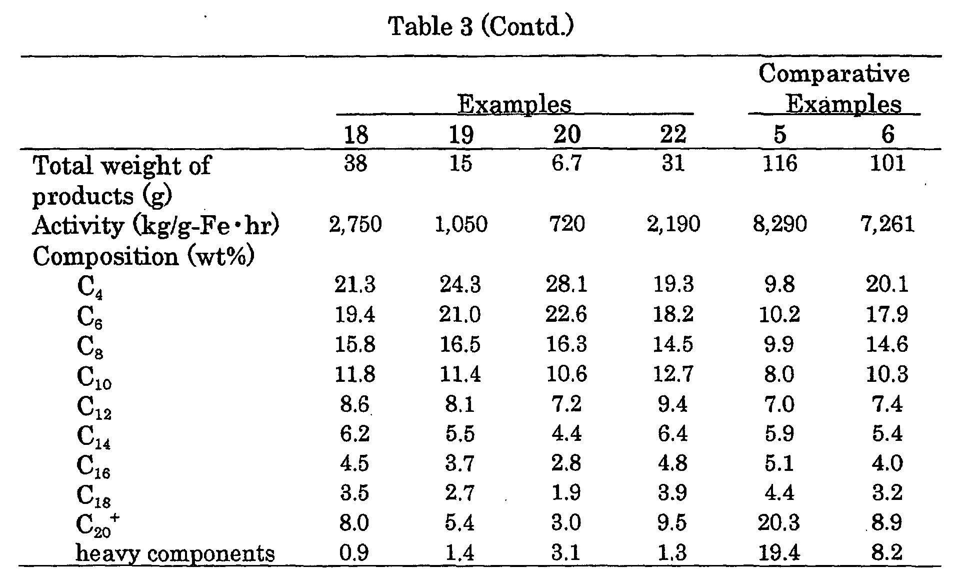

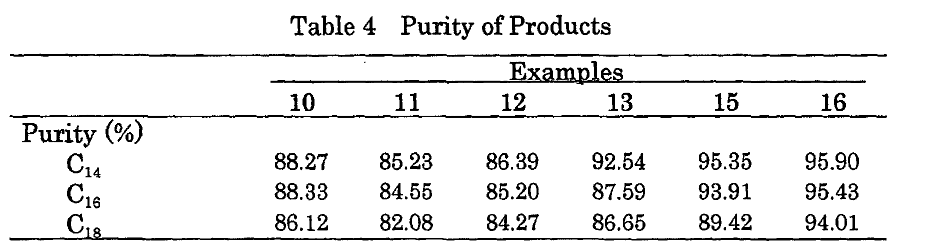

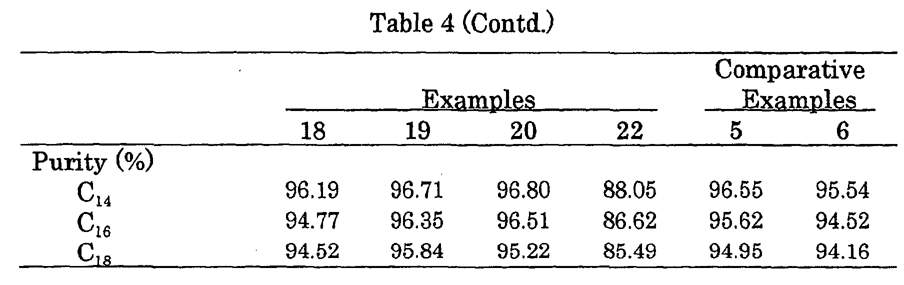

### EXAMPLE 23

### Preparation of 2,6-diacetylpyridine-bis(4-fluoro-2-tolylimine) iron dichloride complex (Compound 49)

### (1) First Step (Imination)

To 2,6-diacetylpyridine (molecular weight: 163.18; 490 mg; 3.00 mmol) placed in a 20-ml round bottom flask, 5 ml of methanol was added and the mixture was stirred. The flask was further added with 4-fluoro-2-toluidine (molecular weight: 125.15; density: 1.126; 1.67 ml; 15.0 mmol) and then with five drops of formic acid. After ten and several minutes, the precipitation of crystals was initiated. After 1.5 hours of the reaction, the solids were separated from the supernatant liquid by filtration and washed with methanol to obtain 2,6-diacetylpyridine-bis(4-fluoro-2-tolylimine) as a ligand (molecular weight:377.44; 788 mg; 2.09 mmol; yield: 69.5%).

The ¹H-NMR chart of the ligand (90 MHz; solvent: CDCl₃; standard: tetramethylsilane (δ0.00)) is shown in Fig. 1 as the NMR chart No.1.

### (2) Second Step (Preparation of iron complex)

Into a 50-ml Schlenk tube, iron (II) chloride tetrahydrate (formula weight: 198.81; 298 mg; 1.50 mmol) was charged and then 10 ml of THF (tetrahydrofuran) was added to the tube to dissolve the iron chloride. Separately, the above 2,6-diacetylpyridine-bis(4-fluoro-2-tolylimine) (formula weight: 377.44; 585 mg; 1.55 mmol) was dissolved into 10 ml of THF in a 50-ml Schlenk tube. Next, the THF solution of 2,6-diacetylpyridine-bis(4-fluoro-2-tolylimine) was added to the THF solution of iron (II) chloride tetrahydrate through a cannula. The cannula was washed with 10 ml of THF, and the washing was added to the mixture. The reaction proceeded immediately, thereby precipitating dark violet crystals. After one hour, the solids were separated from the supernatant liquid by filtration, washed with 15 ml of diethyl ether twice and then dried under reduced pressure to obtain violet crystals of titled 2,6-diacetylpyridine-bis(4-fluoro-2-tolylimine) iron dichloride complex (molecular weight: 504.19; 645 mg; 1.28 mmol; yield: 85.5%).

### EXAMPLE 24

### Preparation of 2,6-diacetylpyridine-bis(5-fluoro-2-tolylimine) iron dichloride complex (Compound 50)

### (1) First Step (Imination)

To 2,6-diacetyl pyridine (molecular weight: 163.18; 490 mg; 3.00 mmol) in a 20-ml round bottom flask, 5 ml of methanol was added and the mixture was stirred. The flask was further added with 5-fluoro-2-toluidine (molecular weight: 125.15; 1,877 mg; 15.0 mmol) and then with five drops of formic acid. After performing the reaction over night, the reaction solution was cooled to-78°C to precipitate crystals. The solids were separated from the supernatant liquid by filtration, washed with 5 ml of hexane and 5 ml of methanol and then dried under reduced pressure to obtain titled 2,6-diacetylpyridine-bis(5-fluoro-2-tolylimine) as a ligand (formula weight:377.44; 643 mg; 1.68 mmol; yield: 55.9%).

The ¹H-NMR chart of the ligand (90 MHz; solvent: CDCl₃; standard: tetramethylsilane (δ0.00)) is shown in Fig. 2 as the NMR chart No.2.

### (2) Second Step (Preparation of iron complex)

Iron (II) chloride tetrahydrate (formula weight: 198.81; 298 mg; 1.50 mmol) was charged into a 50-ml Schlenk tube, and then 10 ml of THF (tetrahydrofuran) was added to the tube to dissolve the iron chloride. Separately, the above 2,6-diacetylpyridine-bis(5-fluoro-2-tolylimine) (molecular weight: 377.44; 585 mg; 1.55 mmol) was dissolved into 10 ml of THF in a 50-ml Schlenk tube. Next, the THF solution of 2,6-diacetylpyridine-bis(5-fluoro-2-tolylimine) was added to the THF solution of iron (II) chloride tetrahydrate through a cannula. The cannula washed with 10 ml of THF, and the washing was added to the mixture. The reaction proceeded immediately, thereby precipitating dark violet crystals. After 40 minutes, the crystals were separated from the supernatant liquid by filtration, washed with 15 ml of diethyl ether twice and then dried under reduced pressure to obtain violet crystals of titled 2,6-diacetylpyridine-bis(5-fluoro-2-tolylimine) iron dichloride complex (molecular weight: 504.19; 719 mg; 1.43 mmol; yield: 95.2%).

### EXAMPLE 25

### Preparation of 2,6-diacetylpyridine-bis(3-fluoro-2-tolylimine) iron dichloride complex (Compound 51)

### (1) First Step (Imination)

To 2,6-diacetylpyridine (molecular weight: 163.18; 490 mg; 3.00 mmol) in a 20-ml round bottom flask, 5 ml of methanol was added and the mixture was stirred. The flask was further added with 3-fluoro-2-toluidine (molecular weight: 125.15; density: 1.093; 1.72 ml; 15.0 mmol) and then with five drops of formic acid. After performing the reaction over night, the reaction solution was cooled to -78°C to precipitate crystals. The obtained solids were separated from the supernatant liquid by filtration, washed with 5 ml of hexane and 5 ml of methanol and then dried under reduced pressure to obtain 2,6-diacetylpyridine-bis(3-fluoro-2-tolylimine) ligand (molecular weight:377.44; 967 mg; 2.56 mmol; yield: 85.3%).

The ¹H-NMR chart of the ligand (90 MHz; solvent: CDCl₃; standard: tetramethylsilane (δ0.00)) is shown in Fig. 3 as the NMR chart No.3.

### (2) Second Step (Preparation of iron complex)

Iron (II) chloride tetrahydrate (formula weight: 198.81; 298 mg; 1.50 mmol) was charged into a 50-ml Schlenk tube, and then 10 ml of THF (tetrahydrofuran) was added to the tube to dissolve the iron chloride. Separately, the above 2,6-diacetylpyridine-bis(3-fluoro-2-tolylimine) (molecular weight: 377.44; 585 mg; 1.55 mmol) was dissolved into 10 ml of THF in a 50-ml Schlenk tube. Next, the THF solution of 2,6-diacetylpyridine-bis(3-fluoro-2-tolylimine) was added to the THF solution of iron (II) chloride tetrahydrate through a cannula. The cannula was washed with 10 ml of THF, and the washing was added to the mixture. The reaction proceeded immediately, thereby precipitating dark violet crystals. After 40 minutes, the solids were separated from the supernatant liquid by filtration, washed with 15 ml of diethyl ether twice and then dried under reduced pressure to obtain violet crystals of titled 2,6-diacetylpyridine-bis(3-fluoro-2-tolylimine) iron dichloride complex (molecular weight: 504.19; 619 mg; 1.22 mmol; yield: 81.9%).

### EXAMPLE 26

### Preparation of 2,6-pyridinedicarboxyaldehyde-bis(4-fluoro-2-tolylimine) iron dichloride complex (Compound 52)

### (1) First Step (Imination)

To 2,6-pyridinedicarboxyaldehyde (molecular weight: 135.12; 490 mg; 3.63 mmol) in a 20-ml round bottom flask, 20 ml of methanol was added and the mixture was stirred. The flask was further added with 4-fluoro-2-toluidine (molecular weight: 125.15; density: 1.126; 1.67 ml; 15.0 mmol) and then with five drops of formic acid. After ten and several minutes, the precipitation of crystals was initiated. After performing the reaction for 2 hours, the solids were separated from the supernatant liquid by filtration, washed with 5 ml of methanol three times to obtain 2,6-pyridinedicarboxyaldehyde-bis(4-fluoro-2-tolylimine) ligand (molecular weight:349.38; 1,238 mg; 3.54 mmol; yield: 97.7%).

The ¹H-NMR chart of the ligand (270 MHz; solvent: CDCl₃; standard: chloroform (δ7.24)) is shown in Fig. 4 as the NMR chart No.4.

### (2) Second Step (Preparation of iron complex)

Iron (II) chloride tetrahydrate (formula weight: 198.81; 310 mg; 1.56 mmol) was charged into a 50-ml Schlenk tube, and then 10 ml of THF (tetrahydrofuran) was added to the tube to dissolve the iron chloride. Separately, the above 2,6-pyridinedicarboxyaldehyde-bis(4-fluoro-2-tolylimine) (molecular weight: 349.38; 557 mg; 1.59 mmol) was dissolved into 10 ml of THF in a 50-ml Schlenk tube. Next, the THF solution of 2,6-pyridinedicarboxyaldehyde-bis(4-fluoro-2-tolylimine) was and added to the THF solution of iron (II) chloride tetrahydrate through a cannula. The cannula was washed with 10 ml of THF, and the washing was added to the mixture. The reaction proceeded immediately, thereby precipitating dark violet crystals. After performing the reaction over night, the solvent was distilled off under reduced pressure to reduce the volume of the reaction solution to 20 ml. The solids were separated from a supernatant liquid by filtration and dried under reduced pressure to obtain violet crystals of titled 2,6-pyridinedicarboxyaldehyde-bis(4-fluoro-2-tolylimine) iron dichloride complex (molecular weight: 476.13; 460 mg; 0.97 mmol; yield: 62.9%).

### EXAMPLE 27

### Preparation of 2,6-pyridinedicarboxyaldehyde-bis(5-fluoro-2-tolylimine) iron dichloride complex (Compound 53)

### (1) First Step (Imination)

To 2,6-pyridinedicarboxyaldehyde (molecular weight: 135.12; 490 mg; 3.63 mmol) in a 20-ml round bottom flask, 20 ml of methanol was added and then the mixture was stirred. The flask was further added with 5-fluoro-2-toluidine (molecular weight: 125.15; 1,877 mg; 15.0 mmol) and then with five drops of formic acid. After ten and several minutes, the precipitation of crystals was initiated. After performing the reaction for 2 hours, the solids were separated from the supernatant liquid by filtration, washed with 5 ml of methanol three times to obtain 2,6-pyridinedicarboxyaldehyde-bis(5-fluoro-2-tolylimine) ligand (molecular weight:349.38; 415 mg; 1.18 mmol; yield: 32.8%).

The ¹H-NMR chart of the ligand (270 MHz; solvent: CDCl₃; standard: chloroform (δ7.24)) is shown in Fig. 5 as the NMR chart No.5.

### (2) Second Step (Preparation of iron complex)

Iron (II) chloride tetrahydrate (formula weight: 198.81; 190 mg; 0.96 mmol) was charged into a 50-ml Schlenk tube, and then 10 ml of THF (tetrahydrofuran) was added to the tube to dissolve the iron chloride. Separately, the above 2,6-pyridinedicarboxyaldehyde-bis(5-fluoro-2-tolylimine) (formula weight: 349.38; 366 mg; 1.04 mmol) was dissolved into 10 ml of THF in a 50-ml Schlenk tube. Next, the THF solution of 2,6-pyridinedicarboxyaldehyde-bis(5-fluoro-2-tolylimine) was added to the THF solution of iron (II) chloride tetrahydrate through a cannula. The cannula was washed with 10 ml of THF, and the washing was added to the mixture. The reaction proceeded immediately, thereby precipitating dark violet crystals. After performing the reaction over night, the solvent was distilled off under reduced pressure to reduce the volume of the reaction solution to 20 ml. The solids were separated from a supernatant liquid by filtration and dried under reduced pressure, thereby obtaining violet crystals of titled 2,6-pyridinedicarboxyaldehyde-bis(5-fluoro-2-tolylimine) iron dichloride complex (molecular weight: 476.13; 270 mg; 0.57 mmol; yield: 59.3%).

### EXAMPLE 28

### Preparation of 2,6-pyridinedicarboxyaldehyde-bis(3-fluoro-2-tolylimine) iron dichloride complex (Compound 54)

### (1) First Step (Imination)

To 2,6-pyridinedicarboxyaldehyde (molecular weight: 135.12; 490 mg; 3.63 mmol) in a 20-ml round bottom flask, 20 ml of methanol was added and then the mixture was stirred. The flask was further added with 3-fluoro-2-toluidine (molecular weight: 125.15; density: 1.093; 1.72 ml; 15.0 mmol) and then with five drops of formic acid. After ten and several minutes, the precipitation of crystals was initiated. After performing the reaction for 2 hours, the solids were separated from a supernatant liquid by filtration, washed with 5 ml of methanol three times to obtain 2,6-pyridinedicarboxyaldehyde-bis(3-fluoro-2-tolylimine) ligand (molecular weight:349.38; 1,074 mg; 3.07 mmol; yield: 84.8%).

The ¹H-NMR chart of the ligand (270 MHz; solvent: CDCl₃; standard: chloroform (δ7.24)) is shown in Fig. 6 as the NMR chart No.6.

### (2) Second Step (Preparation of iron complex)

Iron (II) chloride tetrahydrate (formula weight: 198.81; 190 mg; 0.96 mmol) was charged into a 50-ml Schlenk tube, and then 10 ml of THF (tetrahydrofuran) was added to the tube to dissolve the iron chloride. Separately, the above 2,6-pyridinedicarboxyaldehyde-bis(3-fluoro-2-tolylimine) (formula weight: 349.38; 547 mg; 1.57 mmol) was dissolved into 10 ml of THF in a 50-ml Schlenk tube. Next, the THF solution of 2,6-pyridinedicarboxyaldehyde-bis(3-fluoro-2-tolylimine) was added to the THF solution of iron (II) chloride tetrahydrate through a cannula. The cannula was washed with 10 ml of THF, and the washing was added to the mixture. The reaction proceeded immediately, thereby precipitating dark violet crystals. After performing the reaction over night, the solvent was distilled off under reduced pressure to reduce the volume of the reaction solution to 20 ml. The solids was separated from a supernatant liquid by filtration and dried under reduced pressure, thereby obtaining violet crystals of titled 2,6-pyridinedicarboxyaldehyde-bis(3-fluoro-2-tolylimine) iron dichloride complex (molecular weight: 476.13; 488 mg; 1.02 mmol; yield: 66.8%).

### EXAMPLE 29: Oligomerization of Ethylene

Into a 1-liter autoclave, 250 ml of toluene and 1.0 ml of a toluene solution of polymethylalumoxane (available from Toso Akzo Co., Ltd., concentration: 1 mmol/ml) were charged and then 0.5 ml of a 1-µmol/ml toluene suspension of Compound 49 prepared in Example 23 was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction.

After completion of the reaction, the autoclave was depressurized, and the gaseous components were passed into a wet flow meter where the total volume thereof was measured. Then, the gaseous components were analyzed by gas chromatography to determine composition and quantities thereof. The reaction solution was also analyzed by gas chromatography using n-undecane as internal standard to determine the quantity of α-olefins. The solid components were separated by filtration, dried at 120°C for 12 hours, and then subjected to quantitative analysis. As a result, it was confirmed that the total weight of the reaction product was 205 g. The oligomerization activity per unit weight of iron metal was 14,700 kg/g-Fe-hr. The composition and purity of the reaction product were measured by the same analyzing method as described above. The results are shown in Tables 5 and 6. In the Tables, Cx represents a fraction having a carbon number of x; C⁺₂₀ represents a fraction having a carbon number of 20 or more; and the "heavy components" represent solid polymers obtained by the polymerization.

### EXAMPLE 30

The same procedure as in Example 29 was repeated except that the reaction conducted using Compound 50 prepared in Example 24. The results are shown in Tables 5 and 6.

### EXAMPLE 31

The same procedure as in Example 29 was repeated except that the reaction conducted using Compound 51 prepared in Example 25. The results are shown in Tables 5 and 6.

### EXAMPLE 32

Into a 1-liter autoclave, 250 ml of cyclohexane and 1.0 ml of a cyclohexane solution of polymethylalumoxane (obtained by replacing the solvent of the toluene solution of polymethylalumoxane available from Toso Akzo Co., Ltd., with cyclohexane; concentration: 1 mmol/ml) were charged and then 0.5 ml of a 1-µmol/ml cyclohexane suspension of Compound 50 prepared in Example 24 was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction.

After completion of the reaction, the autoclave was depressurized, and the gaseous components were passed into a wet flow meter where the total volume thereof was measured. Then, the gaseous components were analyzed by gas chromatography to determine composition and quantities thereof. The reaction solution was also analyzed by gas chromatography using n-undecane as internal standard to determine a quantity of α-olefins. The solid components were separated by filtration, dried at 120°C for 12 hours, and then subjected to quantitative analysis. As a result, it was confirmed that the total weight of the reaction product was 151 g. The oligomerization activity per unit weight of iron metal was 10,800 kg/g-Fe-hr. The composition and purity of the reaction product were measured by the same analyzing method as described above. The results are shown in Tables 5 and 6.

### COMPARATIVE EXAMPLE 7

The same procedure as in Example 29 was repeated except that the reaction was conducted by using Compound 61 prepared in Comparative Example 1. The results are shown in Tables 5 and 6.

### EXAMPLE 33

Into a 1-liter autoclave, 250 ml of cyclohexane and 0.6 ml of a toluene solution of tetraisobutyl dialuminoxane (available from Aldrich Co., Ltd., 0.18 mmol in terms of aluminoxane) were charged and then 0.4 ml of a 1-µmol/0.1ml toluene suspension of Compound 49 prepared in Example 23 was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction. After completion of the reaction, the autoclave was depressurized to obtain a reaction solution. The reaction solution was analyzed by gas chromatography using n-undecane as internal standard to determine the quantity of α-olefins. The solid components were separated by filtration, dried at 120°C for 12 hours, and then subjected to quantitative analysis. As a result, it was confirmed that the total weight of the reaction product was 116.5 g. The results are shown in Tables 5 and 6.

### EXAMPLE 34

### (1) Preparation of clays

After charging 2g of organic clay S-BEN (phyllosilicates; available from Kunimine Kogyo Co., Ltd.) into a Schlenk tube, and the tube was evacuated in vacuo and the atmosphere was replaced with nitrogen. Then, 40 ml of toluene was added to the clay to form a suspension. To the suspension was then added 3.33 ml of a toluene solution of tetraisobutyl dialuminoxane (available from Aldrich Co., Ltd.; 0.3 mmol/ml), and the resultant mixture was stirred at 100°C for one hour. The mixture was further mixed with 60 ml of toluene, and then allowed to stand. After the supernatant liquid was decanted off, the precipitate was added with toluene so as to adjust the total volume to 100 ml.

### (2) Preparation of catalyst

To 8 ml of the clay solution prepared in the step (1) (containing 160 mg of clay), 0.2 ml of a 1-µmol/0.1ml toluene suspension of Compound 50 prepared in Example 24 was added. The resultant mixture was stirred for 1.5 hours by a magnetic stirrer.

### (3) Oligomerization of Ethylene

Into a 1-liter autoclave, 250 ml of cyclohexane and 3 ml of a toluene solution of tetraisobutyl dialumoxane (available from Aldrich Co., Ltd.; 0.9 mmol in terms of aluminoxane) were charged and then a whole amount of the catalyst solution prepared in the step (2) was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction. After completion of the reaction, the autoclave was depressurized to obtain a reaction solution. The reaction solution was analyzed by gas chromatography using n-undecane as internal standard to determine the quantity of α-olefins. The solid components were separated by filtration, dried at 120°C for 12 hours, and then subjected to quantitative analysis. As a result, it was confirmed that the total weight of the reaction product was 101.3 g. The results are shown in Tables 5 and 6.

### EXAMPLE 35

### (1) Preparation of clays

After charging 2g of organic clay S-BEN (phyllosilicates; available from Kunimine Kogyo Co., Ltd.) into a Schlenk tube, the tube was evacuated in vacuo and the atomsphere was replaced with nitrogen. Then, 40 ml of toluene was added to the clay to form a suspension. To the suspension was then added 3.33 ml of a toluene solution of tetraisobutyl dialuminoxane (available from Aldrich Co., Ltd.; 0.3 mmol/ml), and the resultant mixture was stirred at 100°C for one hour. The mixture was further added with 60 ml of toluene, and then allowed to stand. After the supernatant liquid was decanted off, the precipitate was added with toluene so as to adjust the total volume to 100 ml.

### (2) Preparation of catalyst

To 8 ml of the clay solution prepared in the step (1) (containing 160 mg of clay), was added 0.2 ml of a 1-*µ*mol/0.1ml toluene suspension of Compound 50 prepared in Example 24. The resultant mixture was stirred for 1.5 hours by a magnetic stirrer.

### (3) Oligomerization of Ethylene

Into a 1-liter autoclave, 250 ml of toluene and 3 ml of a toluene solution of tetraisobutyl dialumoxane (available from Aldrich Co., Ltd.; 0.9 mmol in terms of aluminoxane) were charged and then a whole amount of the catalyst solution prepared in the step (2) was added to the autoclave. Further, 10 g of n-undecane as an internal standard was added to the autoclave, and the resultant mixture was heated to 50°C. After heating, while continuously feeding ethylene into the autoclave so as to maintain an inner pressure of the autoclave at 1.0 MPa, the reaction was conducted at 50°C for 30 minutes. Thereafter, a 1-mol/liter sodium hydroxide aqueous solution was added to stop the reaction. After completion of the reaction, the autoclave was depressurized to obtain a reaction solution. The reaction solution was analyzed by gas chromatography using n-undecane as internal standard to determine the quantity of α-olefins. The solid components were separated by filtration, dried at 120°C for 12 hours, and then subjected to quantitative analysis. As a result, it was confirmed that the total weight of the reaction product was 114.3 g. The results are shown in Tables 5 and 6.

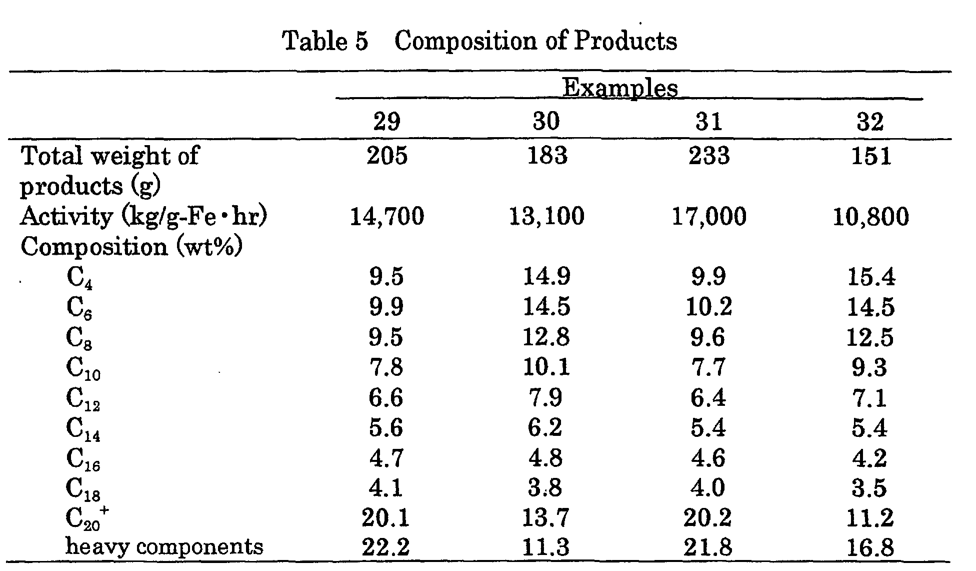

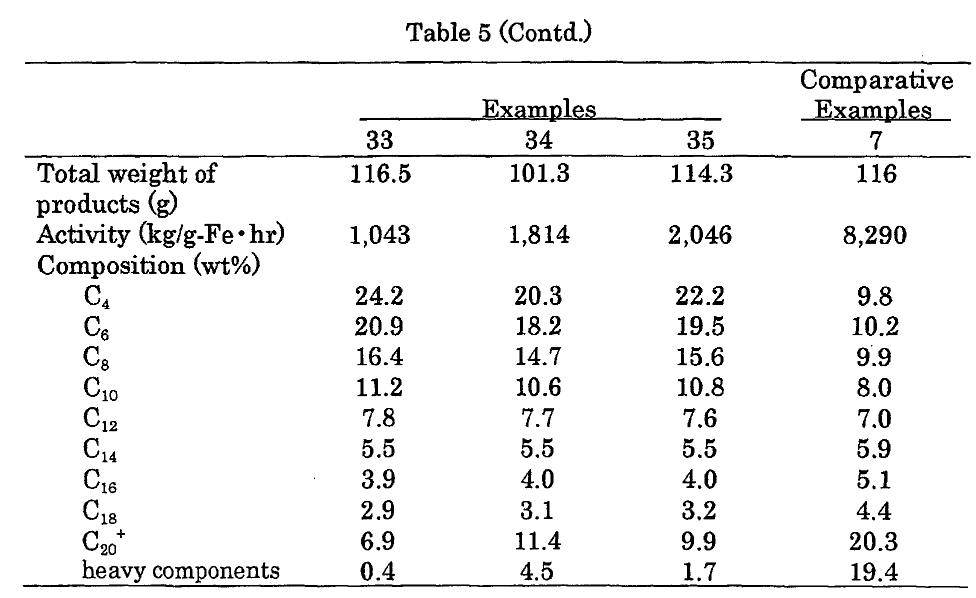

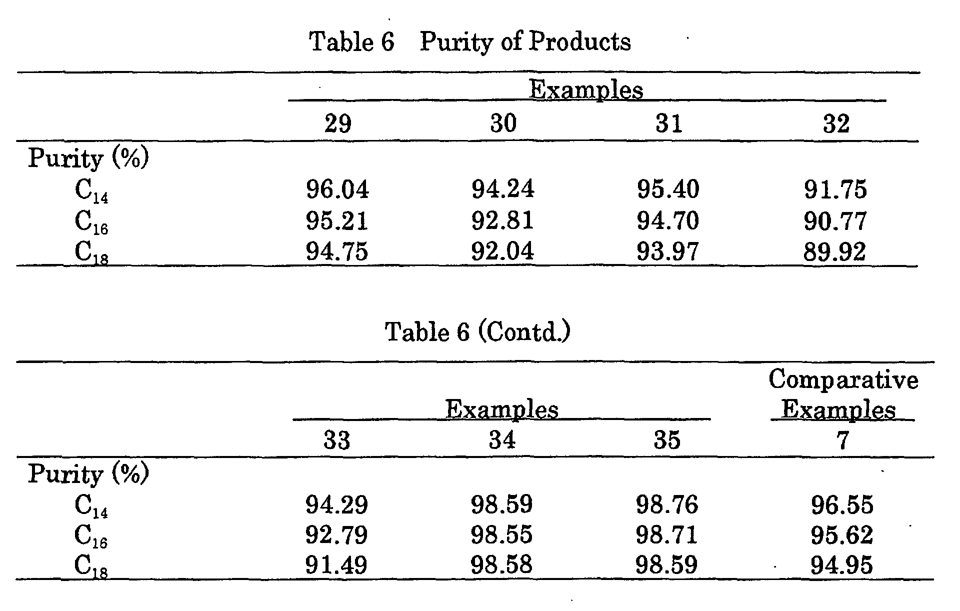

### INDUSTRIAL APPLICABILITY

The catalyst comprising the transition metal compound of the present invention containing a transition metal selected from the group consisting of elements of Groups 8 to 10 of the Periodic Table, exhibits a high ethylene-oligomerization activity, and being capable producing α-olefins from ethylene with less amounts of by-products such as heavy components or waxes with low costs.

## Claims

1. A transition metal compound represented by the general formula (1): 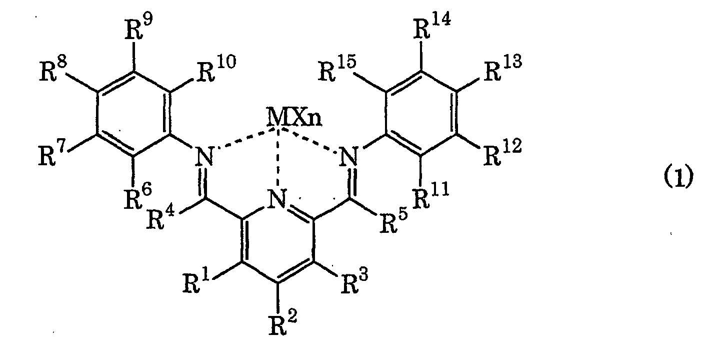 wherein M is a transition metal selected from the group consisting of elements of Groups 8 to 10 of the Periodic Table; R¹ to R³ are independently a hydrogen atom, a halogen atom, a C₁-C₂₀ hydrocarbon group, a C₁-C₂₀ halogenated hydrocarbon group or a hetero atom-containing group, and may be bonded to each other to form a ring; R⁴ and R⁵ are a hydrogen atom or a C₁-C₂₀ hydrocarbon group with the proviso that at least one of R⁴ and R⁵ represents a hydrogen atom, aryl or substituted aryl; R⁶ to R¹⁵ are independently a hydrogen atom, a halogen atom, a C₁-C₂₀ hydrocarbon group or a hetero atom-containing group with the proviso that at least one of R⁶ and R¹⁰ or at least one of R¹¹ and R¹⁵ is a hydrogen atom, and R⁶ to R¹⁵ may be bonded to each other to form a ring; X is a covalent or ionic bonding group, and when a plurality of X groups are present, these groups may be the same or different; and n is a valence of M.

2. The transition metal compound according to Claim 1, wherein the transition metal M is iron or cobalt.

3. A transition metal compound represented by the general formula (2): 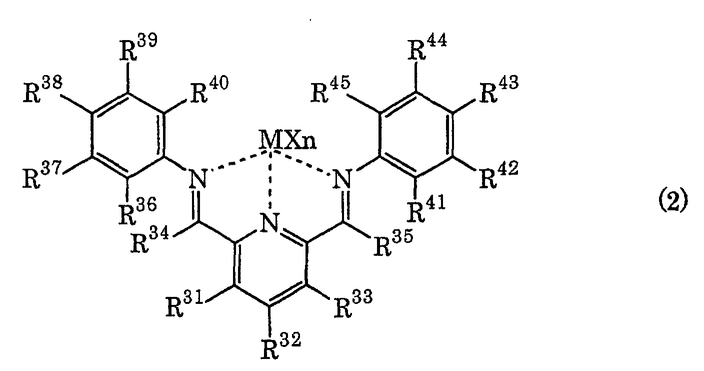 wherein M is a transition metal selected from the group consisting of elements of Groups 8 to 10 of the Periodic Table; R³¹ to R³³ are independently a hydrogen atom, a halogen atom, a C₁-C₂₀ hydrocarbon group, a C₁-C₂₀ halogenated hydrocarbon group or a hetero atom-containing group; R³⁴ and R³⁵ are independently a hydrogen atom or a C₁-C₂₀ hydrocarbon group; R⁴⁰ and R⁴⁵ are a C₁-C₂₀ hydrocarbon group; R³⁶ and R⁴¹ are a hydrogen atom; R³⁷ to R³⁹ are independently a hydrogen atom, a halogen atom or a C₁-C₂₀ hydrocarbon group with the proviso that at least one of R³⁷ to R³⁹ represents a halogen atom; R⁴² to R⁴⁴ are independently a hydrogen atom, a halogen atom or a C₁-C₂₀ hydrocarbon group with the proviso that at least one of R⁴² to R⁴⁴ represents a halogen atom; R³¹ to R³⁵ or R³⁶ to R⁴⁵ may be bonded to each other to form a ring; X is a covalent or ionic bonding group, and when a plurality of X groups are present, these groups may be the same or different; and n is a valence of M.

4. The transition metal compound according to Claim 3, wherein the halogen atom is fluorine.

5. The transition metal compound according to Claim 3 or Claim 4, wherein R³⁷ and R⁴² are fluorine.

6. The transition metal compound according to any one of Claims 3 to 5, wherein R⁴⁰ and R⁴⁵ are methyl.

7. The transition metal compound according to any one of Claims 3 to 6, wherein the transition metal M is iron or cobalt.

8. A catalyst for the production of α-olefins, comprising:
(A) the transition metal compound according to any one of Claims 1 to 7; and
(B) at least one compound selected from the group consisting of an organoaluminum compound (B-1), an ionic compound (B-2) capable of converting the transition metal compound into a cationic transition metal compound, a Lewis acid (B-3), and clay, clay mineral and an ion-exchangeable layer compound (B-4).

9. A catalyst for the production of α-olefins, comprising:
(A) the transition metal compound according to any one of Claims 1 to 7;
(B) at least one compound selected from the group consisting of an organoaluminum compound (B-1), an ionic compound (B-2) capable of converting the transition metal compound into a cationic transition metal compound, a Lewis acid (B-3), and day, clay mineral and an ion-exchangeable layer compound (B-4); and
(C) an organometallic compound.

10. The catalyst according to Claim 8 or Claim 9, wherein the component (B) is alkyl aluminoxane.

11. The catalyst according to Claim 8 or Claim 9, wherein the component (B) is tetraisobutyl dialuminoxane.

12. The catalyst according to Claim 8 or Claim 9, wherein the component (B) is a boron compound.

13. The catalyst according to Claim 8 or Claim 9, wherein the component (B) is phyllosilicate mineral.

14. A process for the production of α-olefins, comprising a step of polymerizing ethylene in the presence of the catalyst according to any one of Claims 8 to 13.
